# EUROPÄISCHE PATENTANMELDUNG

(11) **EP 3 318 293 A1**
(43) Veröffentlichungstag der Anmeldung: **09.05.2018**
(21) Anmeldenummer: 16197294.8
(22) Anmeldetag: 04.11.2016
(51) Int. Cl.: A61M 1/10

(54) **SYSTEM ZUM SICHERN EINER LÖSBAREN VERBINDUNG ZWISCHEN ZWEI ELEMENTEN**

(71) Anmelder: Berlin Heart GmbH, 12247 Berlin (DE)
(72) Erfinder: MATTHES, Michael, 15345 Altlandsberg (DE); LAUTERBACH, Gerhard, 12679 Berlin (DE); WINTERWERBER, Kim Peter, 12357 Berlin (DE); MEISSNER, Maik, 14558 Nuthetal/Saarmund (DE)
(74) Vertreter: Pfenning, Meinig & Partner mbB

(57) **Zusammenfassung**

Die Erfindung betrifft ein System (1) zum Sichern einer lösbaren Verbindung zwischen zwei Elementen (5, 6), insbesondere zwischen zwei Kabeln oder zwischen zwei Hohlkörpern, umfassend:
einen ersten Konnektor (11) und einen zweiten Konnektor (12), der mit dem ersten Konnektor (11) lösbar verbindbar ist,
eine Sicherungshülse (13), die, wenn der erste Konnektor (11) mit dem zweiten Konnektor (12) verbunden ist, durch Verschieben der Sicherungshülse (13) relativ zum ersten Konnektor (11) und relativ zum zweiten Konnektor (12) in eine Sicherungsposition bewegbar ist, in der die Sicherungshülse (13) den ersten Konnektor (11) und den zweiten Konnektor (12) vollständig oder zumindest teilweise in sich aufnimmt, und
eine Rastvorrichtung mit mindestens einem Rastelement (35, 52, 53), wobei die Rastvorrichtung ausgestaltet ist, zwischen der Sicherungshülse (13) in der Sicherungsposition und dem ersten Konnektor (11) und/oder dem mit dem ersten Konnektor (11) verbundenen zweiten Konnektor (12) eine Rastverbindung herzustellen. Die Erfindung betrifft außerdem ein Blutpumpensystem, das das oben genannte System umfasst.

## Beschreibung

Die vorliegende Erfindung betrifft insbesondere ein System zum Sichern einer lösbaren Verbindung zwischen zwei Elementen, beispielsweise zwischen zwei Kabeln oder zwischen zwei Hohlkörpern. Die Erfindung betrifft außerdem ein Blutpumpensystem, das ein solches System beinhaltet.

In verschiedenen Bereichen der Technik ist es erforderlich, lösbare Verbindungen zwischen zwei Elementen, wie etwa zwischen zwei Kabeln oder zwischen zwei Hohlkörpern, zu sichern. Beispielsweise kann ein Ziel des Sicherns einer derartiger Verbindung sein, ein ungewolltes Lösen der Verbindung zu verhindern. Zusätzlich oder alternativ kann es ein Ziel des Sicherns der Verbindung sein, andere Sicherheitsaspekte der Verbindung zu verbessern. Beispielsweise kann es ein Ziel sein, eine elektrische Abschirmung oder eine elektrische Isolation der Verbindung zu verbessern und/oder einen zuverlässigen elektrischen Berührschutz bereitzustellen. Zusätzlich oder alternativ kann es ein Ziel sein, eines oder beide der verbundenen Elemente zu schützen, beispielsweise vor ungewollten Verformungen oder Beschädigungen.

Häufig ist es wichtig, dass das Sichern und das Entsichern der bestehenden Verbindung möglichst schnell und möglichst einfach durchgeführt werden kann. Eine Anforderung kann es auch sein, dass das Sichern und Entsichern der Verbindung rein manuell durchgeführt werden kann, also ohne die Verwendung von Werkzeugen. Außerdem soll die Wahrscheinlichkeit von Fehlbedienungen beim Sichern und Entsichern der Verbindung möglichst klein sein. Eine weitere Zielsetzung kann es zudem sein, die Bedienung des Systems beim Herstellen und beim Lösen der Verbindung zu vereinfachen und zu beschleunigen.

Ein einfaches und schnelles Herstellen und Sichern einer Verbindung wie auch das Entsichern und Lösen der Verbindung ist beispielsweise im Fall von Blutpumpensystem von großer Wichtigkeit. Ein wichtiger Anwendungsfall ist beispielsweise der durch den Patienten oder medizinisch geschultes Personal manuell durchgeführte Austausch einer nichtimplantierten Steuereinheit für eine implantierte Blutpumpe durch eine neue nichtimplantierte Steuereinheit. Hierbei muss die bestehende Verbindung zwischen dem von der implantierten Blutpumpe kommenden transkutanen Kabel und dem von der auszutauschenden Steuereinheit kommenden Kabel zunächst entsichert und gelöst werden. Anschließend muss in möglichst kurzer Zeit eine neue Verbindung zwischen dem von der Blutpumpe kommenden transkutanen Kabel und dem Kabel der neuen Steuereinheit hergestellt und gesichert werden. Eine zuverlässige Sicherung dieser Verbindung (typischerweise eine Steckverbindung) ist bei Blutpumpensystemen (VAD-Systemen) von größter Wichtigkeit, da ein versehentliches oder ungewolltes Lösen dieser Verbindung zum Tod des Patienten führen kann.

Es ist somit eine Aufgabe der vorliegenden Erfindung ein System vorzuschlagen, mit dem eine lösbare Verbindung zwischen zwei Elemente auf möglichst einfache und schnelle Weise gesichert und entsichert werden kann. Ebenso soll ein entsprechendes Blutpumpensystem vorgeschlagen werden, bei dem eine Verbindung, beispielsweise zwischen zwei Kabeln zum Übertragen von Steuer- und Sensorsignalen und zur Energieübertragung für den Antrieb der Blutpumpe oder zwischen zwei Hohlkörpern zum Leiten von Blut, möglichst schnell und einfach gesichert und entsichert werden kann.

Diese Aufgabe wird beispielsweise gelöst durch ein System gemäß dem Hauptanspruch sowie durch ein Blutpumpensystem gemäß dem nebengeordneten Anspruch. Weiterentwicklungen und spezielle Ausführungsbeispiele des Systems wie auch des Blutpumpensystems ergeben sich aus den abhängigen Ansprüchen sowie aus der nachfolgenden Beschreibung und den Figuren.

Das vorgeschlagene System zum Sichern einer lösbaren Verbindung zwischen zwei Elementen umfasst einen ersten Konnektor (Verbinder) und einen zweiten Konnektor (Verbinder), der mit dem ersten Konnektor lösbar verbindbar ist. Das System umfasst außerdem eine Sicherungshülse, die im Folgenden auch kurz als Hülse bezeichnet wird. Die Sicherungshülse ist typischerweise verschiebbar mit dem ersten und/oder dem zweiten Konnektor verbunden. Wenn der erste Konnektor mit dem zweiten Konnektor verbunden ist, ist die Hülse in eine Sicherungsposition bewegbar, beispielsweise durch axiales Verschieben der Sicherungshülse relativ zum ersten Konnektor und dem zweiten Konnektor. Die beiden Elemente, nachfolgend auch als erstes Element und zweites Element bezeichnet, deren lösbare Verbindung durch das System gesichert werden soll, können Bestandteile des Systems sein.

Das hier vorgeschlagene Blutpumpensystem umfasst das hier vorgeschlagene System zum Sichern einer lösbaren Verbindung zwischen zwei Elementen. Das Blutpumpensystem umfasst außerdem eine implantierbare oder nichtimplantierbare Blutpumpe und typischerweise außerdem eine implantierbare oder nicht implantierbare Steuervorrichtung für die Blutpumpe. Das Blutpumpensystem umfasst außerdem beispielsweise ein pumpenseitiges Kabel (beispielsweise als erstes Element) mit einem ersten Kabelende, das mit der Blutpumpe verbindbar ist, und mit einem zweiten Kabelende. Beispielsweise umfasst das Blutpumpensystem außerdem ein steuerseitiges Kabel (beispielsweise als zweites Element) mit einem ersten Kabelende, das mit der Steuervorrichtung verbindbar ist, und mit einem zweiten Kabelende. Die beiden Kabel können beispielsweise zum Übertragen von Steuer- und Sensorsignalen und/oder zur Energieübertragung für den Antrieb der Blutpumpe ausgestaltet sein. Der erste Konnektor des Systems kann in diesem Beispiel mit dem zweiten Kabelende des pumpenseitigen Kabels fest verbunden sein und der zweite Konnektor des Systems kann mit dem zweiten Kabelende des steuerseitigen Kabels fest verbunden sein. In einem typischen Ausführungsbeispiel ist die Blutpumpe implantierbar und die Steuervorrichtung nicht implantierbar. In diesem Fall ist die Steuervorrichtung also für eine extrakorporale Verwendung bestimmt. Das blutpumpenseitige Kabel ist dann zumindest bereichsweise implantierbar und/oder als transkutanes Kabel ausgestaltet. Das zweite Ende des pumpenseitigen Kabels, das steuerseitige Kabel ebenso wie die Konnektoren und die Sicherungshülse sind in diesem Fall typischerweise für eine extrakorporale Verwendung ausgelegt.

Alternativ kann es sich bei den beiden zu verbindenden Elementen auch um Hohlkörper zum Leiten von Blut handeln, wie zum Beispiel ein Pumpeneinlass oder- auslass, ein Verbindungsschlauch oder Verbindungsrohr oder eine Gefäßprothese (Graft). Derartige Hohlkörper, die also einen Fließkanal für eine Flüssigkeit, wie beispielsweise Blut oder eine andere körpereigene oder körperfremde Flüssigkeit, bilden, werden häufig auch als Kanülen bezeichnet. Das System kann somit beispielsweise eine implantierbare oder extrakorporale Kanülenanordnung umfassen oder Teil einer solchen Kanülenanordnung sein.

Entsprechend kann das Blutpumpensystem mit einem solchen System bzw. mit einer solchen Kanülenanordnung als erstes Element einen ersten derartigen Hohlkörper aufweisen, mit einem ersten Ende, das mit der Blutpumpe verbunden oder verbindbar ist, und mit einem zweiten Ende. Als zweites Element kann das Blutpumpensystem einen zweiten derartigen Hohlkörper mit einem ersten Ende und einem zweiten Ende aufweisen. Der erste Konnektor des Systems kann dann mit dem zweiten Ende des ersten Hohlkörpers verbindbar oder (fest oder lösbar) verbunden sein und der zweite Konnektor des Systems mit dem zweiten Ende des zweiten Hohlkörpers verbindbar oder (fest oder lösbar) verbunden sein. Die beiden Konnektoren sind dann typischerweise ausgestaltet, die beiden Hohlkörper fluiddicht miteinander zu verbinden, so dass deren Fließkanäle einen durchgängigen Fließkanal für das Blut bilden.

Im Folgenden werden verschiedene Ausführungsbeispiele für das vorgeschlagene System beschrieben. Da das vorgeschlagene Blutpumpensystem diese Ausführungsbeispiele umfassen kann, ergeben sich gleichzeitig auch entsprechende Ausführungsbeispiele für das vorgeschlagene Blutpumpensystem.

Die Begriffe radial, axial, azimuthal sind bezüglich der Längsachse (Mittelachse) des jeweiligen Teils (Konnektor, Hülse, Element etc.) definiert. Die Sicherungshülse ist relativ zu dem ersten Konnektor und dem hiermit verbundenen zweiten Konnektor in die Sicherungsposition verschiebbar, vorzugsweise durch manuelles Verschieben der Sicherungshülse. Diese Verschiebung erfolgt typischerweise axial, d.h. parallel zur Längsachse der Sicherungshülse und/oder parallel zur den Längsachsen des ersten Konnektors und des zweiten Konnektors. Die Längsachsen des ersten Konnektors und des zweiten Konnektors und der Sicherungshülse sind typischerweise auf einer gemeinsamen Geraden angeordnet, wenn die Verbindung zwischen den beiden Konnektoren besteht. Die axiale Verschiebung erfolgt typischerweise auch relativ zu den beiden (verbundenen) Elementen. Die genannte Sicherungsposition der Sicherungshülse ist relativ zum ersten Konnektor und relativ dem zweiten Konnektor definiert, typischerweise zumindest durch die axiale Position der Sicherungshülse relativ zum ersten Konnektor und relativ zum zweiten Konnektor, wenn beide Konnektoren miteinander verbunden sind.

In der Sicherungsposition nimmt die Sicherungshülse den ersten Konnektor und den zweiten Konnektor jeweils vollständig oder jeweils zumindest teilweise in sich auf bzw. überdeckt den ersten und zweiten Konnektor vollständig oder zumindest teilweise. Die Sicherungshülse definiert einen Hohlraum (ein Lumen oder einen Kanal), der für diesen Zweck geeignet bemessen ist. Typischerweise durchläuft der Hohlraum die Sicherungshülse in axialer Richtung vollständig und durchgängig. Wie weiter unten näher erläutert wird, nimmt die Sicherungshülse in der Sicherungsposition typischerweise zumindest (ggf. einander zugewandte) vordere Enden der beiden Konnektoren vollständig in sich auf sowie vorzugsweise, sofern vorhanden, auch Riegelelemente der Konnektoren und/oder Betätigungselemente der Riegelelemente der Konnektoren.

In einem Ausführungsbeispiel ist die Sicherungshülse durch Verschieben der Sicherungshülse relativ zum ersten Konnektor und/oder relativ zum zweiten Konnektor in eine von der Sicherungsposition verschiedene Halteposition relativ zum ersten Konnektor und/oder relativ zum zweiten Konnektor bewegbar. Vorzugsweise sind beide Konnektoren, wenn sie miteinander verbunden sind, für den Nutzer sichtbar, wenn sich die Sicherungshülse in der Halteposition befindet. Vorzugsweise ist die Sicherungshülse in der Halteposition relativ zu den Konnektoren derart angeordnet, dass beide Konnektoren jeweils ganz oder teilweise, zumindest aber die zwei (ggf. einander zugewandten) vorderen Enden der beiden Konnektoren außerhalb der Sicherungshülse angeordnet und daher sichtbar sind. Auf diese Weise ist es für den Nutzer in vielen Fällen einfacher, die Konnektoren miteinander zu verbinden, besonders dann, wenn für die Verbindung der Konnektoren eine vorgegebene azimutale Ausrichtung der Konnektoren relativ zueinander voraussetzt.

Das System umfasst typischerweise eine Rastvorrichtung mit mindestens einem Rastelement. Außerdem weist die Rastvorrichtung mindestens ein mit dem mindestens einen Rastelement korrespondierendes Gegenrastelement auf, beispielsweise in Form einer Aufnahme oder Haltefläche für das mindestens eine Rastelement. Die Rastvorrichtung ist dazu ausgestaltet, zwischen der Sicherungshülse und dem ersten Konnektor und/oder zwischen der Sicherungshülse und dem zweiten Konnektor eine Rastverbindung herzustellen. Typischerweise ist die Rastvorrichtung derart ausgestaltet, dass die Rastverbindung (vorzugsweise nur dann) herstellbar ist oder besteht, wenn sich die Sicherungshülse relativ zum ersten Konnektor und relativ zum zweiten Konnektor in der Sicherungsposition befindet und wenn außerdem der erste Konnektor mit dem zweiten Konnektor verbunden ist. In einer Variante ist diese Rastverbindung nur zwischen der Sicherungshülse und dem ersten Konnektor vorgesehen. In einer zweiten Variante ist die Rastverbindung nur zwischen der Sicherungshülse und dem zweiten Konnektor vorgesehen. In einer dritten Variante ist die Rastverbindung zwischen der Sicherungshülse einerseits und dem ersten und dem zweiten Konnektor andererseits vorgesehen.

Zusätzlich oder alternativ kann vorgesehen sein, dass die Rastvorrichtung derart ausgestaltet ist, dass eine (weitere) Rastverbindung zwischen der Sicherungshülse und dem ersten Konnektor oder dem zweiten Konnektor herstellbar ist, wenn sich die Sicherungshülse relativ zum ersten Konnektor bzw. relativ zum zweiten Konnektor in der von der Sicherungsposition verschiedenen (relativ zum ersten Konnektor oder relativ zum zweiten Konnektor definierten) Halteposition befindet. Die Sicherungshülse wird dann durch die Rastverbindung in der Halteposition stabilisiert, insbesondere gegenüber ungewollten Verschiebungen in axialer Richtung.

Die genannte Rastverbindung der Sicherungshülse in der Sicherungsposition hat insbesondere den Vorteil, dass sie leicht und schnell herstellbar ist. Die Hülse kann daher sehr einfach und schnell in der Sicherungsposition fixiert werden. Vorzugsweise ist die Rastvorrichtung derart ausgestaltet, dass das mindestens eine Rastelement selbstständig einrastet, wenn die Hülse durch axiales Schieben in die Sicherungsposition bewegt wird. Ein (azimutales) Drehen der Hülse gegenüber den Konnektoren ist hierfür typischerweise nicht oder nur in geringem Maße erforderlich, beispielsweise um eine korrekte (azimutale) Ausrichtung der Rastelemente (beispielsweise gegenüber korrespondierenden Rastflächen bzw. Gegenrastelementen) zur erreichen, sofern erforderlich. Eine solche Drehung beträgt aber typischerweise weniger als 180°, vorzugsweise weniger als 90°. Insbesondere ist die vorgeschlagene Rastverbindung somit insbesondere schneller herstellbar als beispielsweise eine Verbindung mittels eines Gewindes, die typischerweise mehrere volle Umdrehungen der Sicherungshülse gegenüber den Konnektoren erfordern würde.

Durch die Rastverbindung kann die Sicherungshülse in der Sicherungsposition beispielsweise gegenüber ungewollten Verschiebungen in axialer Richtung stabilisiert werden. Nach oder durch Lösen der Rastverbindung kann die Hülse wieder aus der Sicherungsposition heraus bewegt werden, um die Verbindung der Konnektoren zur Entsichern. Zum Lösen der Rastverbindung der Hülse mit dem ersten und/oder zweiten Konnektor kann beispielsweise ein Verschieben der Hülse mit einer vordefinierten axialen Mindestverschiebekraft erforderlich sein, ein Drehen der Hülse um ihre Längsachse und/oder ein radiales Zusammendrücken der Hülse in einer vordefinierten Richtung, wie weiter unten näher erläutert wird.

Typischerweise ist die Sicherungshülse derart ausgestaltet und relativ zum ersten und/oder zweiten Konnektor angeordnet, dass sie zu jeder Zeit zumindest einen axialen Abschnitt des ersten Konnektors und/oder einen axialen Abschnitt des zweiten Konnektors in sich aufnimmt. Typischerweise ist dies zumindest dann der Fall, wenn sich die Sicherungshülse in der Sicherungsposition befindet, typischerweise aber auch dann, wenn sie sich außerhalb der Sicherungsposition befindet, beispielsweise in der Halteposition der Sicherungshülse. Beispielsweise ist die Sicherungshülse am ersten Konnektor mittels eines axialen Anschlag, der am ersten Konnektor oder am ersten Element angeordnet ist, gesichert. In einer hierzu alternativen Variante ist die Sicherungshülse am zweiten Konnektor mittels eines axialen Anschlags, der am zweiten Konnektor oder am zweiten Element angeordnet ist, gesichert. Der Anschlag kann beispielsweise derart angeordnet sein, dass die Hülse, ausgehend beispielsweise von der Sicherungsposition, nur bis zu dem genannten axialen Anschlag relativ zum jeweiligen Konnektor axial verschoben werden kann. Auf diese Weise kann beispielsweise ein ungewolltes Trennen der Hülse vom ersten bzw. zweiten Konnektor vermieden werden.

Bei den zwei genannten Elementen kann es sich beispielsweise jeweils um ein Kabel bzw. um ein Ende eines Kabels handeln. Jedes der Kabel kann eine Au-ßenhülle umfassen, welches mindestens einen Lumen definiert, das sich längs der Außenhülle erstreckt, sowie mindestens einen Leiter, der sich entlang des Kabels und, falls vorhanden, innerhalb der Außenhülle (durch mindestens eine Lumen) erstreckt. Die Außenhülle ist typischerweise aus einem elektrisch isolierenden Material gebildet. Jeder der Leiter kann als elektrischer Leiter zum Übertragen elektrischer Energie und/oder elektrischer Signale oder als optischer Leiter zum Übertragen optischer Signale ausgestaltet sein. Bei den Kabeln kann es sich dann beispielsweise um Kabel für ein Blutpumpensystem handeln, beispielsweise für das hier vorgeschlagene Blutpumpensystem. Derartige Kabel werden häufig auch als "Driveline" des Blutpumpensystems bezeichnet.

Wie oben bereits beschrieben kann es sich bei den zwei genannten Elementen beispielsweise jeweils auch um einen Hohlkörper, wie beispielsweise ein Schlauch, ein Rohr, einen Ein- oder Auslassstutzen einer implantierbaren oder nicht implantierbaren Pumpe, insbesondere einer Blutpumpe, oder eine implantierbare Gefäßprothese (ein sogenannter Graft), handeln, bzw. um ein Ende eines solchen Hohlkörpers. Die Hohlkörper können insbesondere zum Leiten einer Flüssigkeit, wie beispielsweise Blut, ausgestaltet sein, wie im Fall des hier vorgeschlagenen Blutpumpensystems.

Eines oder beide der Elemente können implantierbar sein und können hierfür beispielsweise ganz oder zumindest bereichsweise aus einem biokompatiblen Material bestehen oder mit einem solchen Material vollständig oder zumindest bereichsweise beschichtet sein. Ebenso können die beiden Konnektoren implantierbar sein und hierfür beispielsweise ganz oder zumindest bereichsweise aus einem biokompatiblen Material bestehen oder vollständig oder zumindest bereichsweise mit einem solchen Material beschichtet sein. Auch die Sicherungshülse kann implantierbar sein und hierfür beispielsweise ganz oder zumindest bereichsweise aus einem biokompatiblen Material bestehen oder vollständig oder zumindest bereichsweise mit einem solchen Material beschichtet sein. Als biokompatible Materialien kommen beispielsweise biokompatible Polymere, wie etwa Silikon, und biokompatible metallische Werkstoffe, wie beispielsweise Titan und Nitinol, in Frage.

Die lösbare Verbindung zwischen den beiden Elementen wird vollständig oder zumindest teilweise durch die lösbare Verbindung zwischen dem ersten Konnektor und dem zweiten Konnektor bewirkt. Typischerweise ist zu diesem Zweck der erste Konnektor mit einem der beiden Elemente fest verbunden, welches im Folgenden als das erste Element bezeichnet wird. Typischerweise ist außerdem der zweite Konnektor fest mit dem anderen der beiden Elemente fest verbunden, welches im Folgenden als das zweite Element bezeichnet wird.

Typischerweise umfasst der erste Konnektor und der zweite Konnektor jeweils ein Gehäuse, das typischerweise aus einem metallischen Werkstoff gebildet ist, beispielsweise Messing, Aluminium oder (Edel-)Stahl. Auf diese Weise kann beispielsweise eine hohe Stabilität und Haltbarkeit der Konnektoren erzielt werden. Einer oder beide der Konnektoren können (jeweils ein Griffteil umfassen, das eine äußere (vorzugsweise strukturierte) Grifffläche bildet, um die manuelle Handhabung des Konnektors zu verbessern. Typischerweise ist das Griffteil fest mit dem Gehäuse des jeweiligen (ersten oder zweiten) Konnektors verbunden. Das Griffteil kann ebenfalls aus einem metallischen Werkstoff gebildet sein, beispielsweise Messing, Aluminium (Edel-)Stahl, oder aus einem Polymer, wie etwa Silikon, POM oder PUR.

Jeder der beiden Konnektoren umfasst jeweils das bereits genannte vordere Ende und ein hinteres Ende. Im verbundenen Zustand weist das vordere Ende des ersten Konnektors typischerweise zum zweiten Element und/oder zum zweiten Konnektor und weist typischerweise außerdem das vordere Ende des zweiten Konnektor zum ersten Element und/oder zum ersten Konnektor. Typischerweise sind die Konnektoren über ihre hinteren Enden jeweils mit dem ersten bzw. dem zweiten Element (typischerweise fest oder lösbar) verbunden. Typischerweise überlappen die beiden Konnektoren einander zumindest bereichsweise in axialer Richtung, wenn sie miteinander verbunden sind.

Typischerweise haben die beiden Konnektoren einen möglichst kompakten Aufbau und insbesondere einen möglichst kleinen äußeren Querschnitt, beispielsweise einen runden, vorzugsweise kreisrunden (d.h. rotationssymmetrisch bezüglich Längsachse des ersten bzw. zweiten Konnektors) äußeren Querschnitt.

Beispielsweise können der erste Konnektor und der zweite Konnektor Steckverbinder sein. Die Verbindung zwischen dem ersten Konnektor und dem zweiten Konnektor ist dann also eine Steckverbindung, die durch axiales Zusammen- bzw. Ineinanderschieben der Konnektoren herstellbar ist. Beispielsweise kann der erste Konnektor als ein Stecker und der zweite Konnektor als eine Buchse (wenn der Konnektor z.B. Teil eines Gehäuses ist, z. B. einer Steuereinheit) oder als eine Kupplung (wenn der Konnektor beispielsweise mit einem Kabel fest verbunden ist) ausgestaltet sein. Alternativ kann beispielsweise der erste Konnektor als eine Buchse oder Kupplung und der zweite Konnektor als ein Stecker ausgestaltet sein. Der als Stecker ausgestaltete Konnektor weist an seinem vorderen Ende ein Kupplungselement auf, das beispielsweise stiftförmig oder hülsenförmig ausgestaltet sein kann. Der als Buchse oder Kupplung ausgestaltete Konnektor weist an seinem vorderen Ende ein Aufnahmeteil für das Kupplungselement des Steckers auf. Das Aufnahmeteil kann beispielsweise hülsenförmig ausgestaltet sein. Wenn die beispielsweise derart ausgestalteten Konnektoren miteinander verbunden sind, ist das Kupplungselement typischerweise in einem durch das Aufnahmeteil definierten Aufnahmebereich aufgenommen.

Die Konnektoren können, insbesondere wenn die beiden Elemente als Kabel ausgestaltet sind, korrespondierende Kontaktelemente aufweisen, die zur Übertragung elektrischer und/oder optischer Signale ausgestaltet und derart am ersten und zweiten Konnektor angeordnet sein, dass sie miteinander in Kontakt sind, wenn die beiden Konnektoren miteinander verbunden sind. Beispielsweise kann jedes der Kontaktelemente mit einem der oben beschriebenen Leiter des jeweiligen Kabels verbunden sein. Die Kontaktelemente können beispielsweise im Kupplungselement des als Stecker ausgebildeten Konnektors sowie in dem Aufnahmeteil des als Buchse oder als Kupplung ausgestalteten Konnektors angeordnet sein.

Bei der lösbaren Verbindung zwischen den beiden Konnektoren handelt es sich typischerweise um eine mechanische Verbindung. Typischerweise lässt sich diese Verbindung praktisch beliebig oft herstellen und wieder (zerstörungsfrei) lösen. Vorzugsweise ist die Verbindung zwischen den Konnektoren stabil gegenüber axialer Zugbelastung, beispielsweise durch eine Verriegelung der Verbindung mittels einer Verriegelungsvorrichtung der Konnektoren. Vorzugsweise sind die Konnektoren bzw. die Verriegelungsvorrichtung selbstverriegelnd. Beispielsweise kann der erste Konnektor und/oder der zweite Konnektoren mindestens ein Riegelelement aufweisen, das beispielsweise als Rastelement ausgestaltet sein kann oder ein Rastelement aufweisen kann. Das jeweilige Rastelement kann beispielsweise durch einen Rastarm gegeben sein, der beispielsweise einen radial nach innen oder außen vorspringenden Rastzahn aufweisen kann. Außerdem kann bzw. können der erste und/oder der zweite Konnektor mindestens ein mit dem Riegelelement korrespondierendes Gegenriegelelement aufweisen, beispielsweise in Form einer Aufnahme oder Haltefläche für das mindestens eine Riegelelement. Das mindestens eine Riegelelement ist vorzugsweise aus einem metallischen Werkstoff gebildet. Das Gegenriegelelement ist typischerweise aus einem Kunststoff oder aus einem metallischen Werkstoff gebildet und beispielsweise als eine Vertiefung in dem Gehäuse des ersten oder zweiten Konnektors ausgebildet.

Typischerweise weist der Verriegelungsmechanismus ein (manuell betätigbares) Betätigungselement auf, das mit dem Gehäuse des ersten (alternativ zweiten) Konnektors beweglich (beispielsweise axial verschiebbar) verbunden ist. Das mindestens eine Riegelelement ist beispielsweise mit dem Betätigungselement (erste Variante) oder alternativ mit dem Gehäuse (zweite Variante) des ersten (alternativ zweiten) Konnektors beweglich verbunden, beispielsweise über ein Gelenk. Bei dem (vorzugsweise aus einem metallischen Werkstoff gebildeten) Gelenk kann es sich beispielsweise um einen elastischen Bereich des Betätigungselements oder um ein mit dem jeweiligen Betätigungselement fest verbundenes elastisches Element handeln (erste Variante). Alternativ (zweite Variante) kann es sich bei dem Gelenk um einen Bereich des Gehäuses des ersten (alternativ zweiten) Konnektors oder um ein hiermit fest verbundenes elastisches Element (zweite Variante) handeln.

Beispielsweise ist es möglich, dass das mindestens eine Riegelelement (z.B. mittels des Gelenks) zwischen einer vordefinierten Verriegelungsstellung und einer vordefinierten Entriegelungsstellung bewegbar ist, wobei die Verbindung zwischen den beiden Konnektoren vorzugsweise nur dann (zerstörungsfrei) lösbar ist, wenn sich das mindestens eine Riegelelement in der Entriegelungsstellung befindet. Beispielsweise ist das mindestens eine Riegelelement durch eine (typischerweise manuelle) Betätigung des mindestens einen Riegelelements oder durch eine (typischerweise manuelle) Betätigung eines beweglichen Betätigungselements des jeweiligen Konnektors (wie in der zweiten Variante) bewegbar, beispielsweise von der Verriegelungsstellung in die Entriegelungsstellung zum Entriegeln der Verbindung. Es ist alternativ auch möglich (zum Beispiel in der ersten Variante), dass das mindestens eine Riegelelement nur dann bewegbar ist, beispielsweise von der Verriegelungsstellung in die Entriegelungsstellung, wenn das Betätigungselement (vorzugsweise manuell) betätigt wird, und andernfalls unbeweglich bzw. gesperrt ist, beispielsweise durch das Betätigungselement oder durch ein Sperrelement, das beispielsweise fest mit dem Gehäuse des ersten (alternativ zweiten) Konnektors verbunden sein kann.

Typischerweise (beispielweise in der ersten Variante) wird das mindestens eine Riegelelement in die Verriegelungsstellung bewegt oder in der Verriegelungsstellung gehalten, indem das Sperrelement das Riegelelement in eine Zwangslage drückt (Verriegelungsposition). Diese Verriegelungsautomatik kann beispielsweise bewirken, dass das Kabel des Steckers gezogen werden kann, während die Kupplung (Buchse) fest gehalten wird. Dadurch bleibt das Riegelelement, welches mit dem relativ zum Steckergehäuse frei beweglichen Betätigungselement über ein Gelenk befestigt ist (Variante 1), durch typischerweise kegelförmige, aneinander gleitende Flächenpaare (eine Kegelfläche am Riegelelement, eine Kegelfläche am Sperrelement) in der Verriegelungsstellung. Die Kegelflächen bewirken, dass die Kegelflächen um so mehr gegeneinander drücken, je höher die Zugkraft am Kabel ist. Dadurch werden die axialen Zugkräfte je nach Kegelwinkel radial abgelenkt, so dass höhere Kräfte höhere Verriegelungskräfte bewirken (Pressung zwischen den Kegelflächenpaaren). In dem Betätigungsfall ist es dann typischerweise nicht möglich, das Betätigungselement in die Entriegelungsposition zu bewegen. Das Betätigungselement kann beispielsweise durch (vorzugsweise manuelle Betätigung) zwischen einer Sperrstellung und einer Entsperrstellung bewegbar sein, wobei das Riegelelement nur dann von der Verriegelungsstellung in die Entriegelungsstellung bewegbar ist, wenn sich das Betätigungselement in der Entsperrstellung befindet.

Vorzugsweise ist das mindestens eine Riegelelement oder, sofern vorhanden, das mindestens eine Betätigungselement für das mindestens eine Riegelelement derart angeordnet, dass es, wenn die beiden Konnektoren miteinander verbunden sind, von außen (manuell) nicht zugänglich (und somit auch nicht manuell betätigbar) ist, wenn sich die Hülse in der Sicherungsposition befindet. Das mindestens eine Riegelelement bzw. das zugehörige Betätigungselement sind dann in dem Hohlraum der Hülse angeordnet. Auf diese Weise ist die Verbindung der Konnektoren durch die Hülse vor einer ungewollten Entriegelung geschützt. Vorzugsweise ist das mindestens eine Riegelelement bzw. das Betätigungselement, wenn die beiden Konnektoren miteinander verbunden sind, nur dann von außen (manuell) zugänglich und betätigbar, wenn die Hülse ausreichend weit aus der Sicherungsposition heraus bewegt ist, beispielsweise in die oben beschriebene Halteposition. Dadurch, dass die Hülse in der Sicherungsposition eingerastet ist, wird ein ungewolltes Bewegen der Hülse aus der Sicherungsposition und ein darauf prinzipiell ermöglichtes ungewolltes Entriegeln der Verbindung der Konnektoren verhindert oder zumindest erschwert. Dies ist insbesondere wichtig bei transkutanen VAD-Drivelines. Hier besteht grundsätzlich das Ziel, einen möglichst kleinen Durchmesser des Konnektors (bei der Operation bzw. Implantation wichtig) wie auch ein geringes Gewicht und eine geringe Länge (für Nutzer im Alltag wichtig) des Konnektors zu erreichen. Daher sind die typischerweise im Markt erhältlichen Konnektoren und deren Bedienkräfte relativ klein. Kleine Bedienkräfte erhöhen in der Regel jedoch das Risiko des ungewollten Öffnens der Verbindung.

Vorzugsweise ist das mindestens eine Riegelelement und/oder, sofern vorhanden, das jeweilige Gelenk des Riegelelements oder des Betätigungselements, derart ausgestaltet, zum Beispiels durch eine entsprechende Vorspannung des Riegelelements bzw. des Gelenks, dass sich das Riegelelement in der Verriegelungstellung befindet oder sich selbsttätig in diese hinein bewegt, wenn keine Betätigungskraft oder andere äußere Kraft auf das Riegelelement bzw. auf das Betätigungselement einwirkt. Dieser Effekt kann beispielsweise auch durch ein entsprechend ausgebildetes Federelement des jeweiligen Konnektors bewirkt werden, dass beispielsweise mit dem Riegelelement und dem Gehäuse des Konnektors verbunden ist. Auf diese Weise können die Konnektoren beispielsweise selbstverriegelnd sein.

Wie oben bereits beschrieben, kann der erste oder zweite Konnektor mindestens ein korrespondierendes Gegenriegelelement für das mindestens eine Riegelelement aufweisen, etwa in der Form einer Aufnahme, Haltefläche oder Vertiefung in dem Gehäuse des jeweiligen Konnektors. In diesem Fall befindet sich typischerweise das mindestens eine Riegelelement ganz oder zumindest teilweise in einem gegenseitigen Eingriff mit diesem Gegenriegelelement, wenn sich das Riegelelement in der Verriegelungsstellung befindet und die beiden Konnektoren miteinander verbunden sind. Das mindestens eine Riegelelement und das mindestens eine korrespondierende Gegenriegelelement sind vorzugsweise derart ausgeformt, dass die Verbindung der Konnektoren verriegelt ist, wenn sich das mindestens eine Riegelelement in der Verriegelungsstellung befindet, und nur durch (typischerweise manuelles) Entriegeln der Verbindung, also durch Bewegen des mindestens einen Riegelelements in die Entriegelungsstellung, in der das mindestens eine Riegelelement sich nicht in einem gegenseitigen Eingriff mit dem jeweiligen Gegenriegelelement befindet, (zerstörungsfrei) gelöst werden kann. Wie oben beschrieben, kann dies eine Betätigung eines Betätigungselements voraussetzen. Das Lösen erfolgt dann typischerweise durch axiales Auseinanderziehen der Konnektoren.

Beispielsweise kann es sich bei der Verbindung zwischen den Konnektoren um eine Rastverbindung handeln. Hierfür kann bzw. können der erste Konnektor und/oder der zweite Konnektor Rastelemente bzw. korrespondierende Gegenrastelemente, beispielsweise in Form von Aufnahmen oder Halteflächen für die Rastelemente, aufweisen. Die Rastelemente des ersten und/oder zweiten Konnektors sind typischerweise verschieden von dem mindestens einen Rastelement der oben genannten Rastvorrichtung, welche die Rastverbindung zwischen der Sicherungshülse und dem ersten und/oder zweiten Konnektor herstellt. Beispielweise können die oben beschriebenen Riegelelemente als Rastelemente ausgestaltet sein und entsprechend die genannten Gegenriegelelement als korrespondierende Gegenrastelemente.

Mögliche Beispiele für verriegelbare Konnektoren sind sogenannte Push-Pull-Konnektoren, also Konnektoren die zur Verriegelung einen Push-Pull-Mechanismus aufweisen, wie beispielsweise in EP 2 287 974 B1 beschrieben. Weitere mögliche Varianten von Push-Pull-Konnektoren werden beispielsweise von den Herstellern Fischer Connectors und Lemo angeboten.

Insbesondere im Fall, dass die beiden Elemente Hohlkörper sind, wie beispielsweise Kanülen, können die beiden Konnektoren dazu ausgestaltet sein, die beiden Elemente mittels eines Kraftschlusses und/oder eines Reibschlusses zu verbinden. Bilden die beiden Hohlkörper Fließkanäle, wie etwa für Blut oder andere Flüssigkeiten, sie sind die beiden Konnektoren in der Regel dazu ausgestaltet, die beiden Hohlkörper fluiddicht miteinander zu verbinden, so dass diese einen durchgängigen Fließkanal bilden.

Beispielsweise kann der der erste Konnektor zumindest teilweise innerhalb des Fließkanals des ersten Elements angeordnet sein oder einen Teil des Fließkanals des ersten Elements bilden. Sind beide Elemente miteinander verbunden, kann der erste Konnektor beispielsweise auch teilweise innerhalb des Fließkanals des zweiten Elements angeordnet sein. Beispielsweise kann der erste Konnektor am zweiten Ende des ersten Elementes teilweise in den Fließkanal des ersten Elements eingeschoben sein und teilweise aus diesem herausragen. Der erste Konnektor kann beispielsweise mit dem ersten Element fest verbunden und beispielsweise einteilig mit dem ersten Element ausgebildet sein. Beispielsweise kann der erste Konnektor durch einen axialen Endabschnitt des ersten Elements an dessen zweiten Ende gebildet sein, an dem das erste Element beispielsweise einen reduzierten Außendurchmesser aufweisen kann.

Sind beide Elemente miteinander verbunden, so ist der erste Konnektor dann typischerweise in den Fließkanal des zweiten Elements bzw. Hohlkörpers eingeschoben. In diesen Fällen Fall bildet also der erste Konnektor somit einen Teil des Fließkanals des Systems.

Der der erste Konnektor kann beispielsweise als ein Rohrelement, wie beispielsweise eine (Zentrier-)Hülse, ausgestaltet sein. Das Rohrelement kann beispielsweise derart dimensioniert ist, dass es in den ersten und/oder den zweiten Hohlkörper des ersten bzw. zweiten Elements eingeschoben werden kann, beispielsweise in einen Fließkanal des ersten bzw. zweiten Hohlkörpers.

Der zweite Konnektor kann als ein korrespondierendes Klemmelement, beispielsweise als Schelle oder Schlitzhülse, ausgestaltet sein. Der zweite Konnektor ist dann typischerweise derart dimensioniert, dass er das zweite Ende des zweiten Elements sowie das darin eingeschobene vordere Ende des ersten Konnektors umgreifen kann, um auf diese Teile eine Klemmkraft einzuleiten. Hierfür kann vorgesehen sein, dass der zweite Konnektor in einen gespannten Zustand überführt werden kann, um eine (beispielsweise radial nach innen oder alternativ nach außen) auf das zweite Element einwirkende Klemmkraft auszuüben und somit eine fluiddichte Klemmverbindung zwischen dem zweiten Element und dem ersten Konnektor herzustellen. Beispielweise kann weist der zweite Konnektor zur Herstellung und/oder zum Stabilisieren des gespannten Zustands einen Schraubverschluss mit einer Spannschraube und einem korrespondierenden Gewindeteil aufweisen. Alternativ könnte der zweite Konnektor auch einen Rastverschluss mit entsprechenden Rastelementen oder ein elastisches Federelement umfassen.

Natürlich ist auch eine umgekehrte Rollenverteilung des ersten und zweiten Konnektors als die oben beschriebene möglich.

Beispielsweise kann der als Rohrelement ausgestaltete erste oder zweite Konnektor ein Gegenrastelement der Rastvorrichtung, beispielsweise eine Rastfläche, insbesondere eine Ringnut, aufweisen. Zusätzlich oder alternativ kann auch der als Klemmelement ausgestaltete zweite oder erste Konnektor (z.B. die Schelle), ein Gegenrastelement der Rastvorrichtung aufweisen, beispielsweise eine Rastfläche, insbesondere eine Ringnut.

Das mindestens eine Rastelement der Rastvorrichtung kann genau ein Rastelement oder auch mehr als ein Rastelement umfassen. Wenn im Folgenden der besseren Lesbarkeit halber von "einem Rastelement" oder von "dem Rastelement" die Rede ist, ist damit "mindestens ein Rastelement des mindestens einen Rastelements der Rastvorrichtung" bzw. "das mindestens eine Rastelement des mindestens einen Rastelements der Rastvorrichtung" gemeint. Sofern mehrere Rastelemente vorhanden sind, können sich die jeweils gemachten Angaben daher entweder auf nur ein einziges Rastelement der Rastvorrichtung beziehen oder auch auf mehrere oder auf alle Rastelemente der Rastvorrichtung.

Die Rastvorrichtung kann mindestens ein Gegenrastelement umfassen. Beispielsweise kann für jedes Rastelement ein korrespondierendes Gegenrastelement vorgesehen sein. Ist mittels der Rastvorrichtung eine der beschriebenen Rastverbindungen hergestellt, so können sich beispielsweise eines oder mehrere des mindestens einen Rastelements jeweils mit einem Gegenrastelement in einem gegenseitigen Eingriff befinden. Beispielsweise kann das Rastelement das jeweilige Gegenrastelement hintergreifen. Beispielsweise können die Rastelemente beweglich, flexibel und/oder elastisch ausgestaltet sein, während die Gegenrastelemente beispielsweise unbeweglich und starr ausgestaltet sein können. Eine umgekehrte Konfiguration ist ebenso möglich.

Beispielsweise kann ein Rastelement der Rastvorrichtung ausgestaltet sein,
- auf einer Oberfläche des ersten Konnektors einzurasten oder
- auf einer Oberfläche des zweiten Konnektors einzurasten oder
- auf einer Oberfläche der Sicherungshülse einzurasten,
wenn sich die Sicherungshülse in der Sicherungsposition befindet, oder, zusätzlich oder alternativ hierzu, wenn sich die Sicherungshülse in der Halteposition befindet. In diesen Ausführungsbeispielen können die genannten Oberflächen beispielsweise durch die oben genannten Gegenrastelemente gebildet sein, beispielsweise in Form von Aufnahmebereichen oder Rastflächen für das jeweilige Rastelement. Derartige Gegenrastelemente können beispielsweise auch durch eine geeignete Form der Außenkontur des ersten oder zweiten Konnektors oder durch eine geeignete Form der Innenkontur der Hülse gebildet sein. Das jeweilige Gegenrastelement kann beispielsweise durch einen Hinterschnitt, eine Nut, eine Rille, eine Vertiefung, ein Steg, eine Ausbuchtung oder ähnliches auf der jeweiligen Oberfläche gebildet sein. Es ist möglich, die dass die Oberfläche bzw. das Gegenrastelement durch das Gehäuse des Konnektors oder ein weiteres (beispielsweise ringförmiges) Element, wie beispielsweise ein Griffteil, gebildet ist, das mit dem jeweiligen Konnektor bzw. dessen Gehäuse oder mit der Hülse fest verbunden ist.

Beispielsweise können das Rastelement und das jeweilige Gegenrastelement derart ausgeformt sein, dass die Sicherungshülse durch axiales Verschieben der Sicherungshülse aus der Sicherungsposition oder der Halteposition heraus bewegbar ist, wenn eine auf die Sicherungshülse einwirkende axiale Verschiebekraft einen vordefinierten Schwellwert überschreitet. Beispielsweise können das Rastelement oder das jeweilige Gegenrastelement hierfür (jeweils) eine angewinkelte Steigflächen aufweisen, die derart angewinkelt ist, dass der gegenseitige Eingriff des Rastelements und des Gegenrastelements sich löst, wenn die Sicherungshülse mit dieser Verschiebekraft aus der Sicherungsposition bzw. der Halteposition herausgeschoben wird.

Beispielsweise kann ein Rastelement, mehrere Rastelemente oder jedes Rastelement der Rastvorrichtung
- ein Bestandteil des ersten Konnektor oder
- ein Bestandteil des zweiten Konnektors oder
- ein Bestandteil der Sicherungshülse sein,
und/oder fest mit dem ersten Konnektor, dem zweiten Konnektor bzw. der Sicherungshülse verbunden sein, so dass sich das jeweilige Rastelement hiervon nicht löst, wenn die Sicherungshülse aus der Sicherungsposition (oder der Halteposition) heraus bewegt wird.

Die Rastvorrichtung kann beispielweise einen Träger für das Rastelement bzw. die Rastelemente umfassen. Der Träger kann beispielsweise hülsenförmig ausgestaltet sein. Der Träger kann ein Teilbereich der Sicherungshülse sein oder ein in die Sicherungshülse integriertes Element sein. Beispielsweise kann die feste Verbindung zwischen dem jeweiligen Rastelement und dem ersten Konnektor, dem zweiten Konnektor bzw. der Sicherungshülse durch Verkleben hergestellt sein.

Die Sicherungshülse und das mit ihm fest verbundene mindestens eine Rastelement können auch durch ein integrales Spritzgussteil gebildet sein, beispielsweise durch ein 2K-Spritzgussteil, wie weiter unten beschrieben wird.

Ein Rastelement, mehrere Rastelemente oder jedes Rastelement der Rastvorrichtung kann beispielsweise beweglich sein und/oder ganz oder zumindest bereichsweise elastisch ausgestaltet sein. Das jeweilige Rastelement kann beispielsweise aus einem metallischen oder einem polymeren Material gebildet sein.

Beispielsweise kann ein Rastelement, mehrere Rastelemente oder jedes Rastelement der Rastvorrichtung als ein elastisches Ringelement ausgestaltet sein.

Zum Beispiel kann oder können das Rastelement bzw. die Rastelemente jeweils als eine ringförmig gewickelte Spiralfeder oder als ein Axialsicherungsring oder als ein O-Ring ausgestaltet sein. Ein solches elastisches Ringelement umläuft typischerweise die Längsache der Sicherungshülse, des ersten Konnektors oder des zweiten Konnektors vollständig (azimuthal). Beispielsweise kann mindestens eine mit diesem Rastelement korrespondierende (in sich geschlossene) Ringnut auf der Innenfläche der Hülse, auf der äußeren Oberfläche des ersten Konnektors oder auf der äußeren Oberfläche des zweiten Konnektors vorgesehen sein, welche typischerweise die Längsache der Sicherungshülse, des ersten Konnektors oder des zweiten Konnektors vollständig (azimuthal) umläuft. Beispielsweise kann das elastische Ringelement teilweise innerhalb einer derartigen Ringnut auf der inneren Oberfläche der Hülse angeordnet und dort beispielsweise durch Eigenspannung dauerhaft befestigt sein und in eine korrespondierende Ringnut auf der äußeren Oberfläche des ersten oder zweiten Konnektors eintauchen, wenn sich die Hülse in der Sicherungsposition (oder in der Halteposition) befindet. Eine umgekehrte Konfiguration ist natürlich ebenso möglich. Bei der Spiralfeder kann es sich beispielsweise um eine Spiralfeder der in der EP 0890758 A2 ("coiled spring") oder der in der EP 1468192 B1 ("Schraubenfeder") beschriebenen Art handeln.

Ferner kann bzw. können beispielsweise ein Rastelement, mehrere Rastelemente oder jedes Rastelement der Rastvorrichtung eine Ausbuchtung oder einen Vorsprung aufweisen, die bzw. der von einer inneren Oberfläche der Sicherungshülse ausgehend radial nach innen vorspringt. Es ist umgekehrt möglich, dass ein Rastelement, mehrere Rastelemente oder jedes Rastelement der Rastvorrichtung eine Ausbuchtung oder einen Vorsprung aufweist bzw. aufweisen, die bzw. der von einer äußeren Oberfläche des ersten oder zweiten Konnektors ausgehend radial nach außen vorspringt.

Die genannte Ausbuchtung bzw. der genannte Vorsprung kann beispielsweise jeweils als eine Noppe, als eine Rippe oder als ein Steg ausgestaltet sein.

Die Sicherungshülse kann einen hülsenförmigen Hauptkörper umfassen. Die genannte Ausbuchtung bzw. der genannte Vorsprung, die bzw. der beispielsweise von der Innenfläche des Hauptkörpers radial nach innen vorspringt, kann weicher als der hülsenförmige Hauptkörper der Sicherungshülse sein. Beispielsweise können der Hauptkörper und die Ausbuchtung bzw. der Vorsprung aus verschiedenen Materialen gebildet sein, die sich in ihrer Festigkeit unterscheiden. Beispielsweise kann die Hülse ein 2K-Spritzgussteil sein.

In einem Ausführungsbeispiel ist vorgesehen, dass die Rastvorrichtung zwei oder mehr Rastelemente aufweist. Hiervon weisen mindestens zwei Rastelemente jeweils eine Ausbuchtung auf, die von einer inneren Oberfläche der Sicherungshülse radial nach innen vorspringen. Die Sicherungshülse hat einen (entspannten) Ausgangszustand, den sie beispielsweise dann einnimmt, wenn keine äußere Kraft, insbesondere keine radiale Kompressionskraft, auf sie einwirkt. Die beiden Ausbuchtungen nehmen in dem Ausgangszustand der Sicherungshülse einen ersten Abstand voneinander ein. Der erste radiale Abstand ist in einer ersten radialen Richtung definiert und derart bemessen, dass die beiden Ausbuchtungen in der Oberfläche des ersten Konnektors oder der Oberfläche des zweiten Konnektors einrasten oder eingerastet sind, wenn sich die Sicherungshülse im Ausgangszustand und relativ zu dem ersten Konnektor und dem mit dem ersten Konnektor verbundenen zweiten Konnektor in der Sicherungsposition befindet. Beispielsweise sind die beiden Ausbuchtungen dann in jeweils eine korrespondieren Aufnahme auf der Oberfläche des ersten oder zweiten Konnektors eingetaucht oder hintergreifen korrespondierende Ausbuchtungen auf der Oberfläche des ersten oder zweiten Konnektors.

Die Sicherungshülse ist in diesem Beispiel durch eine in einer zweiten radialen Richtung, die von der ersten radialen Richtung verschieden ist, auf die Sicherungshülse einwirkende radiale Kompressionskraft (welche eine vorgegebene Stärke aufweist) in einen (gespannten) Zwischenzustand verformbar. Diese Kompressionskraft kann beispielsweise manuelle Zusammendrücken der Hülse bewirkt werden. Hierzu kann die Hülse beispielsweise auf ihrer Außenseite entsprechend markierte Bereiche aufweisen. Die genannte Verformung der Hülse ist vorzugsweise elastisch, so dass sich die Sicherungshülse von selbst wieder in den Ausgangszustand zurück verformt, sobald die radiale Kompressionskraft nicht mehr auf sie einwirkt.

Die beiden Ausbuchtungen nehmen in dem Zwischenzustand der Sicherungshülse einen in der ersten radialen Richtung definierten zweiten Abstand voneinander ein. Der zweite Abstand ist größer als der erste Abstand und derart bemessen, dass die beiden Ausbuchtungen in der Oberfläche des ersten Konnektors und/oder der Oberfläche des zweiten Konnektors nicht eingerastet sind (sie tauchen dann also beispielsweise nicht in die oben genannten Aufnahmen ein bzw. hintergreifen nicht die oben genannten Ausbuchtungen), wenn sich die Sicherungshülse in ihrem Zwischenzustand und sich gleichzeitig relativ zu dem ersten Konnektor und dem mit dem ersten Konnektor verbundenen zweiten Konnektor in der (axial definierten) Sicherungsposition befindet. Die Hülse rastet also in der Sicherungsposition nicht ein, wenn sie sich in ihrem Zwischenzustand befindet, weil die beiden Ausbuchtungen hierfür einen zu großen radialen Abstand von der Oberfläche des ersten bzw. zweiten Konnektor einnehmen.

Solange sich die Hülse in dem Zwischenzustand befindet, zum Beispiel durch anhaltendes Zusammendrücken, kann die Hülse relativ zum ersten und zweiten Konnektor axial in Sicherungsposition geschoben werden, ohne dass eine Rastverbindung zwischen der Hülse und den Konnektoren besteht oder hergestellt wird. Sobald sich die Hülse in der Sicherungsposition befindet und dort (selbständig) in ihren Ausgangszustand zurückkehrt, etwa durch Beenden des Zusammendrückens, rastet die beiden genannten Rastelemente mit ihren Ausbuchtung auf der Oberfläche des ersten oder des zweiten Konnektors ein. Zum Lösen dieser Rastverbindung kann die Hülse durch Zusammendrücken in den Zwischenzustand überführt und gehalten werden und im Zwischenzustand durch axiales Verschieben aus der Sicherungsposition wieder heraus bewegt werden.

Die Sicherungshülse bzw. deren hülsenförmiger Hauptkörper kann einen Querschnitt, insbesondere eine Innenkontur, aufweisen, die beispielsweise im Ausgangszustand eine ovale (oder alternativ kreisrunde, d.h. rotationssymmetrisch bezüglich der Längsachse) Form hat und im Zwischenzustand beispielsweise eine kreisrunde (alternativ ovalen) Form. Vorzugsweise ist ein kleinster Innendurchmesser der Hülse im Zwischenzustand größer als ein größter Außendurchmesser des ersten Konnektors und/oder als ein größter Außendurchmesser des zweiten Konnektors. Typischerweise haben der erste Konnektor und/oder der zweite Konnektor einen (äußeren) Querschnitt, also eine Außenkontur, der bzw. die kreisrund ist (d.h. rotationssymmetrisch bezüglich der Längsachse).

Beispielsweise kann die Rastvorrichtung mindestens einen flexiblen Rastarm aufweisen. Der Rastarm weist beispielsweise ein Rastelement der Rastvorrichtung in Form einer radial nach innen vorspringenden Ausbuchtung auf. Diese Ausbuchtung wird im Folgenden auch als Rastzahn bezeichnet. Der Rastarm erstreckt sich typischerweise in axialer Richtung, also im Wesentlichen parallel zur Längsachse der Hülse. Typischerweise ist der Rastarm mit der Sicherungshülse fest verbunden.

Beispielsweise kann die Oberfläche des ersten Konnektors oder die Oberfläche des zweiten Konnektors mindestens eine Stufe aufweisen, die eine Rastfläche für den mindestens einen Rastarm bildet. Befindet sich die Hülse in der Sicherungsposition, hintergreift der Rastarm mit seinem Rastzahn die Stufe bzw. die Rastfläche. Die Stufe bzw. die Rastfläche können beispielsweise durch einen (endständigen) Bund oder einen (endständigen) Steg des Konnektors gebildet sein oder durch eine Nut oder eine Rille. Befindet sich die Hülse in der Sicherungsposition und ist der Rastzahn in der Oberfläche des ersten oder zweiten Konnektors eingerastet, so besteht die Rastverbindung zwischen der Sicherungshülse und dem ersten bzw. zweiten Konnektor.

Der erste Konnektors und/oder der zweiten Konnektor kann bzw. können mindestens eine Entriegelungssteigfläche aufweisen, die beispielsweise auf der (äußeren) Oberfläche des Konnektors angeordnet ist und beispielsweise an die durch die Stufe gebildete Rastfläche angrenzt. Typischerweise ist die Entriegelungssteigfläche auf einer Rückseite der Stufe angeordnet. Die Rastarme gleiten bei einer (azimutalen) Drehung der Hülse in der Sicherungsposition über die Entriegelungssteigflächen und werden dabei radial auseinandergespreizt. Typischerweise gleiten hierbei die radial nach innen gerichteten Ausbuchtungen bzw. Rastzähne der Rastarme über die Entriegelungssteigflächen.

Typischerweise weist der Rastarm eine Steigfläche auf, die auf einer Vorderseite des Rastzahns angeordnet ist, und eine Haltefläche, die auf einer der Vorderseite abgewandten Rückseite des Rastzahns angeordnet ist. Allgemein ist die Vorderseite des Rastzahns dem (typischerweise freitragenden) Kopfende des Rastarms zugewandt und ist die Haltefläche des Rastzahns dem (typischerweise an die Hülse bzw. an einen Träger angrenzenden) Fußende des Rastarms zugewandt.

Die Haltefläche, die vorzugsweise eben ist, kann gegenüber einer Bezugsebene, die senkrecht zur Längsachse der Sicherungshülse ist, angewinkelt sein. Vorzugsweise liegt der Winkel, der zwischen der Haltefläche und der Bezugsebene eingeschlossen wird, zwischen 30° und 60°. Zusätzlich oder alternativ kann die Steigfläche, die vorzugsweise eben ist, gegenüber der genannten Bezugsebene angewinkelt sein. Vorzugsweise liegt der Winkel, der zwischen der Steigfläche und der Bezugsebene eingeschlossen wird, zwischen 30° und 60°. Durch die Wahl des Winkels der Steigfläche kann beispielsweise eine (manuelle) Betätigungskraft für das Einrasten des Rastarms vorgegeben werden und auf diese Weise beispielsweise die (manuelle) Betätigungskraft definiert werden, die aufgebracht werden muss, um die Hülse in die Sicherungsposition hinein zu schieben und die Rastverbindung zwischen der Hülse und dem ersten und/oder zweiten Konnektor herzustellen. Entsprechend kann durch die Wahl des Winkels der Haltefläche eine Betätigungskraft für das Entrasten des Rastarms vorgegeben werden und auf diese Weise beispielsweise die (manuelle) Betätigungskraft definiert werden, die erforderlich ist, die Hülse aus der Sicherungsposition heraus zu schieben und die Rastverbindung zwischen der Hülse und dem ersten und/oder zweiten Konnektor zu lösen.

Das System kann außerdem ein formflexibles Schutzelement für das erste Element und/oder für das zweite Element aufweisen. Typischerweise definiert das Schutzelement einen Kanal für das erste und/oder zweite Element, in den das erste und/oder zweite Element also zumindest abschnittsweise aufgenommen werden kann, beispielsweise dann, wenn sich die Sicherungshülse in der Sicherungsposition befindet. Der Kanal des Schutzelementes bildet beispielsweise einen Teil oder eine (typischerweise koaxial verlaufende) Fortsetzung des Hohlraums der Sicherungshülse.

Das Schutzelement kann beispielsweise als ein Knickschutz und/oder als Schneidschutz für das erste bzw. zweite Element ausgestaltet sein. Dies kann insbesondere dann vorteilhaft sein, wenn das jeweilige Element als Kanüle, z.B. als Gefäßprothese aus einem Graftmaterial, ausgestaltet ist. Das Schutzelement ist typischerweise mechanisch stabiler als das jeweils zu schützende Element ausgestaltet.

Das Schutzelement ist typischerweise fest mit der Sicherungshülse verbunden. Sie kann auch als ein Teil der Sicherungshülse ausgebildet sein. Beispielsweise kann die Sicherungshülse einen Hauptkörper aufweisen, der typischerweise hülsenförmig ausgestaltet ist und den genannten Hohlraum der Sicherungshülse teilweise oder vollständig bildet. Der Hauptkörper ist vorzugsweise stabil (d.h. starr und fest) ausgestaltet und kann mit dem Schutzelement fest oder lösbar verbunden sein. Typischerweise weist der Hauptkörper mindestens ein Rastelement der Rastvorrichtung auf.

Die Sicherungshülse bzw. ihr Hauptkörper kann mit dem Schutzelement beispielsweise stoffschlüssig verbunden sein, beispielsweise mit einem vorderen Ende des Schutzelements oder beispielsweise mit einem vordersten der weiter unten beschriebenen Segmente (falls das Schutzelement segmentartg aufgebaut ist).

Das Schutzelement kann wie das in der Europäischen Patentanmeldung EP 16 16 4527.0 beschriebene Schutzelement ausgestaltet sein. Beispielsweise kann das Schutzelement also eine Mehrzahl von Segmenten umfassen, die in einer Abfolge nebeneinander angeordnet sind. Jedes der Segmente definiert dann einen Teilabschnitt des Kanals des Schutzelements.

Die Segmente können ganz oder zumindest teilweise aus einem elastisch verformbaren Polymer gebildet sein, beispielsweise aus einem Silikon, einem Silikonelastomer oder einem Polyurethan. Das Polymer (z.B. weiches Silikon) kann beispielsweise Shore A 30-90 aufweisen. Die Segmente können beispielsweise Spritzgussteile sein.

Das Schutzelement kann mindestens ein Verbindungselement aufweisen, welches die Segmente miteinander verbindet. Das Verbindungselement oder die Verbindungselemente kann bzw. können beispielsweise stoffschlüssig mit den Segmenten verbunden sein.

Das mindestens eine Verbindungselement kann als mindestens ein kabelförmiges Element ausgestaltet sein, beispielsweise mit rundem oder flachem Querschnitt. Das mindestens eine kabelförmige Element kann mit zwei oder mit mehr als zwei der Segmente oder mit jedem der Segmente direkt verbunden sein, beispielsweise stoffschlüssig. Das mindestens eine kabelförmige Element kann zwei oder mehr derartige kabelförmige Elemente umfassen, wobei die kabelförmigen Elemente in einer Umfangsrichtung um das Schutzelement voneinander beabstandet angeordnet sind.

Die Segmente können derart miteinander verbunden sein, dass die Segmente relativ zueinander beweglich sind. Beispielsweise können die Segmente relativ zueinander um eine Längsachse des Kanals drehbar sein, beispielsweise um mindestens 3° oder um mindestens 5°.

Im Fall des beschriebenen segmentartigen Aufbaus kann das Schutzelement gekürzt werden, indem zwei in der Abfolge benachbarte Segmente voneinander getrennt werden, beispielsweise indem mindestens ein oder ein jedes Verbindungselement von Verbindungselementen des Schutzelements, welches die beiden benachbarten Segmente miteinander verbindet, mittels eines manuell geführten Schneidinstruments, beispielsweise mittels eines Skalpells, durchschnitten wird.

Weitere mögliche Ausführungsbeispiele für das Schutzelement sind der europäischen Patentanmeldung EP 16 16 4527.0 zu entnehmen.

In Ausführungsbeispielen, in denen die Sicherungshülse ein derartiges Schutzelement aufweist, weist die Sicherungshülse vorzugsweise ein Rastelement der Rastvorrichtung auf. Dieses kann als ein elastisches Ringelement ausgestaltet sein, welches den Hohlraum der Sicherungshülse ringförmig umläuft. Das Rastelement ist vorzugsweise als eine ringförmig gewickelte Spiralfeder oder als ein Axialsicherungsring oder als ein O-Ring ausgestaltet.

Beispielsweise kann der oben genannte (typischerweise hülsenförmige) Hauptkörper der Sicherungshülse aus Titan oder einem anderen Metall oder Polymer geformt sein kann. Der Hauptkörper kann beispielsweise mit dem Schutzelement verklebt sein. In den Hauptkörper kann beispielsweise eine weitere, beispielsweise aus Polyoxymethylen (POM) oder einem anderen Polymer gebildete Hülse eingeschraubt sein. Diese kann auf einer Innenfläche eine Ringnut zur Aufnahme des oben genannten elastischen Ringelements (z. B. eine ringförmig gewickelte Spiralfeder) aufweisen.

Der (hülsenförmige) Hauptkörper der Sicherungshülse kann alternativ auch aus einem Polymer geformt sein, das im Vergleich zum Schutzelement vorzugsweise relativ hart ist (vorzugsweise Shore B-C), beispielsweise ein entsprechend hartes Silikon. Die Ringnut für das elastische Ringelement kann beispielsweise auch direkt auf dem (hülsenförmigen) Hauptkörper bzw. auf dessen inneren Oberfläche, angeordnet sein. Das Schutzelement kann mit diesem Hauptkörper verklebt sein.

Das elastische Ringelement kann in seiner (kräftefreien) Ausgangsform beispielsweise einen elliptischen Querschnitt aufweisen. Beispielsweise kann eine ringförmig gewickelte Spiralfeder des Hersteller Bal Seal Engineering, Inc. als elastisches Ringelement dienen.

Als Gegenrastelement bzw. Rastfläche für das elastische Ringelement kann der erste Konnektor oder der zweite Konnektor eine entsprechende Ringnut aufweisen, beispielsweise wenn diese als Rohrelement bzw. als Klemmelement ausgestaltet sind, wie bereits oben beschrieben worden ist.

Vorteilhafterweise kann das Schutzelement zusammen mit der Sicherungshülse schnell und sicher über das jeweilige Element, beispielsweise eine Kanüle geschoben werden, ohne dabei das Element bzw. die Kanüle zu verletzen. Die Rastverbindung zwischen der Sicherungshülse und dem ersten bzw. zweiten Konnektor in der Sicherungsposition fixiert typischerweise gleichzeitig auch das Schutzelement. Vorzugsweise, beispielsweise durch das beschriebene elastische Ringelement als Rastelement, kann die Rastverbindung durch eine genügend große axiale Verschiebekraft hergestellt bzw. wieder gelöst werden, ohne dass eine Drehbewegung erforderlich ist. Zudem wird eine sehr kompakte Bauweise ermöglicht.

Entsprechend kann auch das hier vorgeschlagene System als die in der europäischen Patentanmeldung EP 16 16 4527.0 beschriebene Kanülenanordnung ausgestaltet sein, Teil einer solchen Kanülenanordnung sein oder eine solche Kanülenanordnung beinhalten. Ferner kann das hier vorgeschlagene Blutpumpensystem als das in der europälschen Patentanmeldung EP 16 16 4527.0 beschriebene Blutpumpensystem ausgestaltet sein.

Im Folgenden wird das vorgeschlagene System und das vorgeschlagene Blutpumpensystem anhand von in den Figuren 1 bis 29B schematisch dargestellten speziellen Ausführungsbeispielen des Systems, des Blutpumpensystems oder einzelner Komponenten hiervon näher erläutert. Es zeigt
- Figur 1A:: schematische Darstellung eines Systems hier vorgeschlagener Art als Bestandteil eines Blutpumpensystems hier vorgeschlagener Art in einer verbundenen und gesicherten Konfiguration,
- Figur 1B:: das in Figur 1A gezeigte System und Blutpumpensystem in einer verbundenen und entsicherten Konfiguration,
- Figur 1C:: das in Figuren 1A und 1B gezeigte System und Blutpumpensystem in einer getrennten Konfiguration,
- Fign. 2A und 2B:: den ersten Konnektor und den zweiten Konnektor des in Figur 1 gezeigten Systems gemäß einem ersten Ausführungsbeispiel (Beispiel 1) in perspektivischen Darstellungen,
- Fign. 3A und 3B:: die Sicherungshülse des in Figur 1 gezeigten Systems gemäß Beispiel 1, in einer perspektivischen Darstellung bzw. in einer Schnittdarstellung,
- Fign. 4A und 4B: die Konnektoren und die Sicherungshülse gemäß Beispiel 1 in einer verbundenen und gesicherten Konfiguration in Schnittdarstellungen, wobei Figur 4B einen Teilbereich von Figur 4A vergrößert darstellt,
- Figur 5: die Konnektoren und die Sicherungshülse gemäß Beispiel 1 in einer verbundenen und entsicherten Konfiguration in einer Schnittdarstellung,
- Fign. 6A bis 6C: die Konnektoren bzw. einen der Konnektoren und die Sicherungshülse gemäß Beispiel 1 in einer getrennten Konfiguration in Schnittdarstellungen, wobei Figur 6C einen Teilbereich von Figur 6B vergrößert darstellt,
- Fign. 7A und 7B:: den ersten Konnektor und den zweiten Konnektor des in Figur 1 gezeigten Systems gemäß einem zweiten Ausführungsbeispiel (Beispiel 2) in perspektivischen Darstellungen,
- Fign. 8A bis 8C:: die Sicherungshülse des in Figur 1 gezeigte Systems gemäß Beispiel 2, wobei Fig. 8A eine perspektivische Darstellung der Hülse zeigt und Fign. 8B und 8C Schnittdarstellungen zweier möglicher Varianten der Hülse zeigen,
- Fign. 9A bis 9D: die Konnektoren und die Sicherungshülse gemäß Beispiel 2 in einer verbundenen und gesicherten Konfiguration in Schnittdarstellungen, wobei Figuren 9B und 9C jeweils einen Teilbereich von Figur 9A vergrößert darstellen,
- Figur 10: die Konnektoren und die Sicherungshülse gemäß Beispiel 2 in einer verbundenen und entsicherten Konfiguration in einer Schnittdarstellung,
- Fign. 11A und 11B: die Sicherungshülse des in Figur 1 gezeigte Systems gemäß einem dritten Ausführungsbeispiel (Beispiel 3), wobei Fig. 11A eine perspektivischen Schnittdarstellung der Hülse zeigt und Fig. 11B eine seitliche Ansicht auf einen Längsschnitt durch die Hülse,
- Fign. 12A und 12B: Ansichten auf Querschnittflächen der Hülse gemäß Beispiel 3 entlang der in Figur 11B gekennzeichneten und mit C-C bezeichneten Schnittebene, wobei 12A die Hülse in einem gespannten Zwischenzustand und 12B die Hülse in einem entspannten Ausgangszustand zeigt,
- Fign. 13A und 13B: Ansichten auf Querschnittflächen der Hülse gemäß Beispiel 3 entlang der in Figur 11B gekennzeichneten und mit D-D bezeichneten Schnittebene, wobei 13A die Hülse in einem entspannten Ausgangszustand und 13B die Hülse in einem gespannten Zwischenzustand zeigt,
- Fign. 14A und 14B: die Konnektoren und die Sicherungshülse gemäß Beispiel 3 in einer verbundenen und gesicherten Konfiguration in Schnittdarstellungen, wobei Figur 14B einen Teilbereich von Figur 14A vergrößert darstellt,
- Figur 15: die Konnektoren und die Sicherungshülse gemäß Beispiel 3 in einer verbundenen Konfiguration, in der sich die Hülse zwischen der Sicherungsposition und der Halteposition befindet, in ein Schnittdarstellung,
- Fign. 16A bis 16C: die Konnektoren und die Sicherungshülse gemäß Beispiel 3 in einer getrennten Konfiguration, als Schnittdarstellungen oder perspektivische Darstellung, wobei Fig. 16B einen vergrößerten Teilbereich des Systems ein einer Schnittdarstellung zeigt,
- Figur 17: den ersten Konnektor und die Sicherungshülse gemäß Beispiel 3 in einer getrennten Konfiguration, in der sich der Sicherungshülse in einer Halteposition und in dem entspannten Ausgangszustand befindet in einer als perspektivische Schnittdarstellung,
- Fign. 18A und 18B: die Sicherungshülse des in Figur 1 gezeigte Systems gemäß einem vierten Ausführungsbeispiel (Beispiel 4), wobei Fig. 18A eine Seitenansicht und Fig. 18B eine perspektivische Ansicht der Hülse zeigt,
- Fign. 19A und 19B: die Konnektoren und die Sicherungshülse gemäß Beispiel 4 in einer verbundenen und gesicherten Konfiguration, in Schnittdarstellungen, wobei Figur 19B einen Teilbereich von Figur 19A vergrößert darstellt,
- Fign. 20A und 20B: die Konnektoren und die Sicherungshülse gemäß Beispiel 4 in einer verbundenen und entsicherten Konfiguration, in Schnittdarstellungen, wobei Figur 20B einen Teilbereich von Figur 20A vergrößert darstellt,
- Fign. 21A und 21B: die Konnektoren und die Sicherungshülse gemäß Beispiel 4 in einer getrennten Konfiguration, wobei Fig. 21A eine Seitenansicht des Systems und Fig. 21B eine perspektivische Ansicht des Systems zeigt,
- Fig. 22A: die Sicherungshülse des in Figur 1 gezeigte Systems gemäß einem fünften Ausführungsbeispiel (Beispiel 5) in einer perspektivischen Schnittdarstellung,
- Fig. 22B: einen in die Sicherungshülse gemäß Beispiel 5 integrierten Träger,
- Fign. 23A und 23B: die Konnektoren und die Sicherungshülse gemäß Beispiel 5 in einer verbundenen und gesicherten Konfiguration in Schnittdarstellungen, wobei Figur 23B einen Teilbereich von Figur 23A vergrößert darstellt,
- Fign. 24A und 24B: die Konnektoren und die Sicherungshülse gemäß Beispiel 5 in einer verbundenen und entsicherten Konfiguration, in Schnittdarstellungen, wobei Figur 24B einen Teilbereich von Figur 24A vergrößert darstellt,
- Figur 25A: schematische Darstellung eines Systems hier vorgeschlagener Art als Bestandteil eines Blutpumpensystems hier vorgeschlagener Art in einer getrennten Konfiguration,
- Figur 25B: das in Figur 25A gezeigte System und Blutpumpensystem in einer verbundenen und entsicherten Konfiguration,
- Figur 25C: das in Figuren 25A und 25B gezeigte System und Blutpumpensystem in einer verbundenen und gesicherten Konfiguration,
- Fign. 26A und 26B: Beispiele von Elementen und Konnektoren für das in Figuren 25A bis 25C gezeigte System und Blutpumpensystem, jeweils in einem verbundenen nicht gesicherten Zustand in einer Längsschnittdarstellung und in einer perspektivischen Darstellung,
- Fign. 27A und 27B: ein Beispiel einer Sicherungshülse für das in Figuren 25A bis 25C gezeigte System und Blutpumpensystem, in einer perspektivischen Darstellung und in einer Längsschnittdarstellung,
- Figur 28: die in Figuren 26A bis 27B gezeigten Komponenten in einer perspektivischen Darstellung, wobei die Sicherungshülse entlang des zweiten Elements axial verschoben wird, und
- Fign. 29A und 29B: die in Figuren 26A bis 28 gezeigten Komponenten in einer perspektivischen Darstellung und in einer Längsschnittdarstellung, wobei die Sicherungshülse sich in der Sicherungsposition befindet.

Gleiche oder einander entsprechende Merkmale sind mit den gleichen Bezugszeichen versehen.

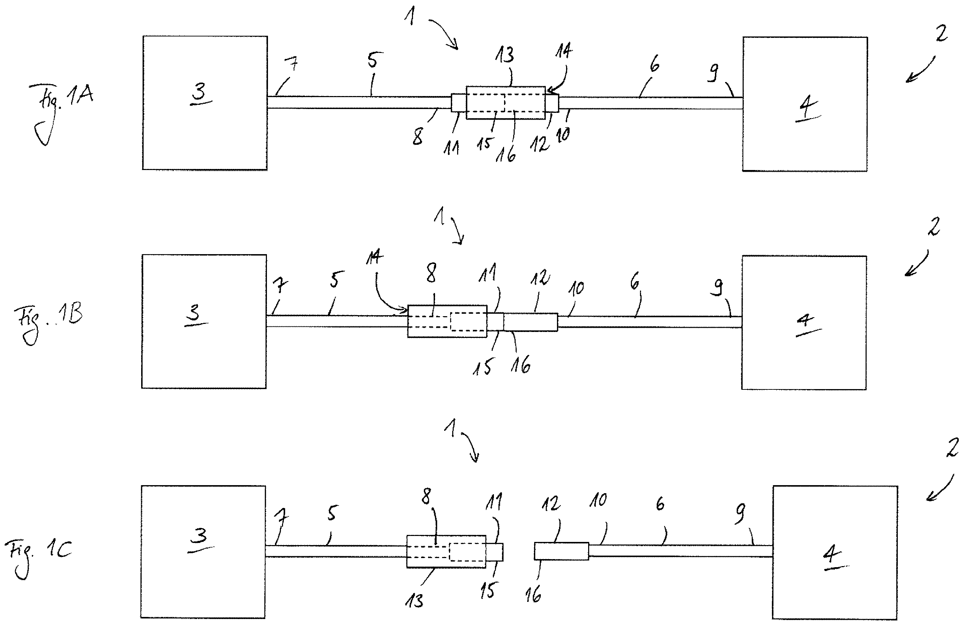

Figuren 1A bis 1C zeigen jeweils eine schematische Darstellung eines Systems 1 zum Sichern einer lösbaren Verbindung zwischen zwei Elementen hier vorgeschlagener Art als Bestandteil eines Blutpumpensystems 2 hier vorgeschlagener Art.

Das in Figuren 1A bis 1C gezeigte Blutpumpensystem 2 umfasst außerdem eine implantierbare Blutpumpe 3 und eine extrakorporale (nicht implantierbare) Steuervorrichtung 4 für die Blutpumpe 3. Das Blutpumpensystem 2 umfasst ein erstes Element 5 in Form eines pumpenseitigen Kabels mit einem ersten Ende 7, das mit der Blutpumpe 3 verbindbar ist, und mit einem zweiten Ende 8. Das Blutpumpensystem 2 umfasst außerdem ein zweites Element 6 in Form eines steuerseitiges Kabels mit einem ersten Ende 9, das mit der Steuervorrichtung 4 verbindbar ist, und mit einem zweiten Ende 10. Steuervorrichtung 4 ist dazu eingerichtet, über die beiden Elemente (Kabel) 5 und 6 elektrische und/oder optische Signale an die Blutpumpe 3 zu senden oder von dieser zu empfangen. Hierzu können die Kabel elektrische und/oder optische Leiter umfassen, die sich jeweils längs des jeweiligen Kabels erstrecken. Die Kabel umfassen typischerweise jeweils eine Außenhülle, die ein oder mehrere Lumen für die Leiter definieren. Die Außenhüllen sind typischerweise aus einem elektrisch isolierenden Material gebildet.

Das System 1 ist dazu eingerichtet, die beiden Elemente (Kabel) 5 und 6 miteinander lösbar zu verbinden, um die Signalübertragung zwischen Blutpumpe 3 und Steuervorrichtung 4 zu ermöglichen. Hierzu umfasst das System 1 einen ersten Konnektor 11 und einen zweiten Konnektor 12, der mit dem ersten Konnektor 11 lösbar verbindbar ist. In Figuren 1A und 1B sind die beiden Konnektoren 11, 12 miteinander verbunden und in Figuren 1C voneinander getrennt. Der erste Konnektor 11 des Systems 1 ist mit dem zweiten Ende 8 des ersten Elements 5 (pumpenseitiges Kabel) fest verbunden. Der zweite Konnektor 12 ist mit dem zweiten Ende 10 des zweiten Elements 12 (steuerseitiges Kabel) fest verbunden.

Das erste Element 11 (blutpumpenseitiges Kabel) ist bereichsweise implantierbar und als transkutanes Kabel ausgestaltet. Hierfür kann das erste Elemente 11, insbesondere die Außenhülle des ersten Elements 11, ganz oder zumindest bereichsweise aus einem biokompatiblen Material bestehen oder mit einem solchen Material vollständig oder zumindest bereichsweise beschichtet sein. Als biokompatible Materialien kommen beispielsweise biokompatible Polymere, wie etwa Silikon in Frage.

Das System 1 umfasst außerdem eine Sicherungshülse 13 bzw. Hülse 13. Die Sicherungshülse 13 ist verschiebbar mit dem ersten Konnektor 11 verbunden. Wenn der erste Konnektor 11 mit dem zweiten Konnektor 12 verbunden ist, wie in Figur 1A gezeigt ist, ist die Hülse 3 durch manuelles axiales Verschieben der Sicherungshülse 13 relativ zum ersten Konnektor 11 und dem zweiten Konnektor 12 in eine Sicherungsposition bewegbar. Die Sicherungsposition ist relativ zum ersten Konnektor 11 und relativ dem zweiten Konnektor 12 durch die axiale Position der Sicherungshülse relativ zum ersten Konnektor 11 und relativ zum zweiten Konnektor 12 definiert, wenn beide Konnektoren 11, 12 miteinander verbunden sind.

Das zweite Ende 8 des ersten Elements 11, das zweite Element 12 (steuerseitiges Kabel) ebenso wie die Konnektoren 11, 12 und die Sicherungshülse 13 des Systems 1 sind in diesem Beispiel für eine extrakorporale Verwendung ausgelegt.

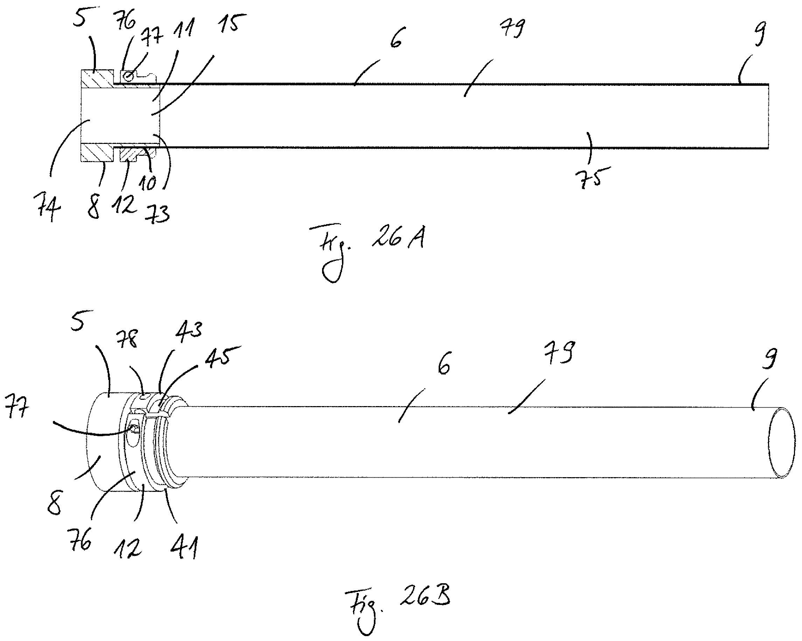

In einem alternativen Ausführungsbeispiel des Systems 1 und des Blutpumpensystems könnte es sich bei den beiden zu verbindenden Elementen 11, 12 des Blutpumpensystems 2 auch um Hohlkörper zum Leiten von Blut handeln. Beispielsweise könnte des erste Element 5 ein Pumpenauslass des Blutpumpe 3 sein und das zweite Element 6 eine Gefäßprothese, welche beispielsweise mit einem Blutgefäß, zum Beispiel einer Aorta oder einer Vene des Patienten verbunden ist. Die Konnektoren 11, 12 können dann ebenfalls zum Leiten von Blut und implantierbar ausgestaltet sein. Ein derartiges Ausführungsbeispiel wird weiter unten anhand von Figuren 26A bis 29B näher beschrieben.

Wie in Figur 1A gezeigt ist, nimmt die Sicherungshülse 13 in der Sicherungsposition den ersten Konnektor 11 und den zweiten Konnektor 12 jeweils teilweise in sich auf und überdeckt so den ersten und zweiten Konnektor 11, 12 teilweise. Die Sicherungshülse 13 definiert zu diesem Zweck einen Hohlraum 14, der die Sicherungshülse 14 in axialer Richtung vollständig und durchgängig durchläuft. Insbesondere nimmt die Sicherungshülse 13 in der Sicherungsposition einander zugewandte vordere Enden 15, 16 der beiden Konnektoren 11, 12 vollständig in sich auf sowie, sofern vorhanden, auch Riegelelemente der Konnektoren und/oder Betätigungselemente der Riegelelemente der Konnektoren.

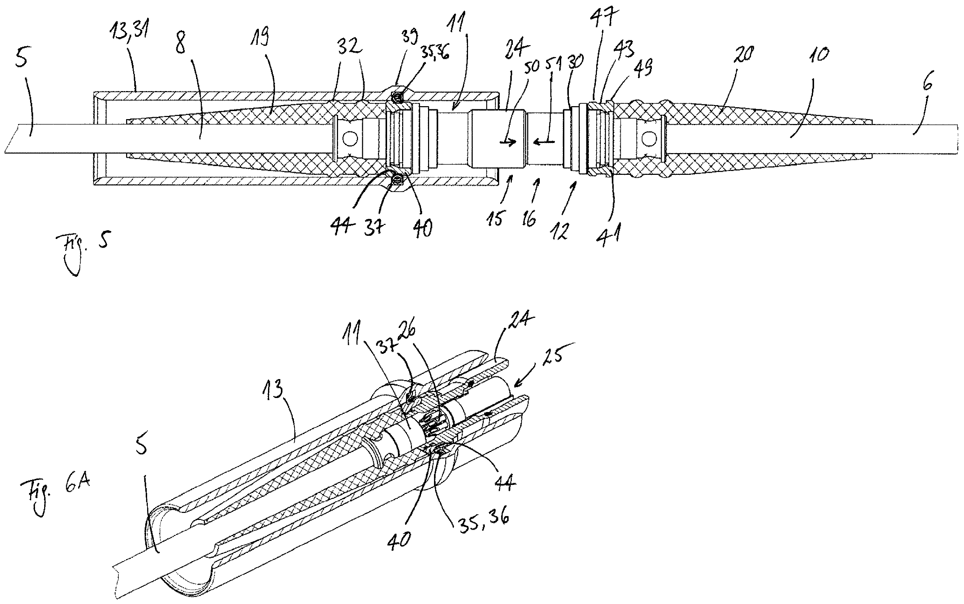

Die Sicherungshülse 13 ist durch manuelles Verschieben der Sicherungshülse 13 relativ zum ersten Konnektor 11 und/oder relativ zum zweiten Konnektor 12 in eine von der Sicherungsposition verschiedene Halteposition relativ zum ersten Konnektor 11 und/oder relativ zum zweiten Konnektor bewegbar, wie beispielsweise in Figuren 1B und 1C dargestellt ist. Wie in Figur 1B gezeigt ist, sind die beiden Konnektoren 11, 12, wenn sie miteinander verbunden sind, für den Nutzer sichtbar, wenn sich die Sicherungshülse 13 in der Halteposition befindet. Vorzugsweise ist die Sicherungshülse 13 in der Halteposition relativ zu den Konnektoren 11, 12 derart angeordnet, dass beide Konnektoren 11, 12 jeweils ganz oder teilweise, zumindest aber die zwei einander zugewandten vorderen Enden 15, 16 der beiden Konnektoren 11, 12 außerhalb der Sicherungshülse angeordnet und daher sichtbar sind. Auf diese Weise ist es für den Nutzer insbesondere dann, wenn für die Verbindung der Konnektoren 11, 12 eine vorgegebene azimutale Ausrichtung der Konnektoren relativ zueinander eingehalten werden muss, einfacher, die Konnektoren miteinander zu verbinden. Beispielsweise können hierfür Markierungen an den Konnektoren 11, 12 angeordnet sein, welche eine korrekte Ausrichtung der Konnektoren 11, 12 zueinander erkennbar macht, wenn die Sicherungshülse 13 sich in der Halteposition befindet (siehe beispielsweise Markierungen 50, 51 in Figur 5 oder entsprechend gekennzeichnete Markierungen bei den anderen Beispielen).

In dem in Figuren 1A bis 1C gezeigten Beispiel ist eine Verschiebung der Hülse 13 aus der Sicherungsposition in Richtung des ersten Elementes 5 bzw. in Richtung der Blutpumpe 3 gezeigt, so dass die Halteposition auf Seite der Blutpumpe 3 angeordnet ist. Es wäre aber auch umgekehrt möglich, dass eine Verschiebung der Hülse 13 aus der Sicherungsposition in Richtung des zweiten Elementes 5 bzw. in Richtung der Steuervorrichtung 4 vorgesehen ist. Die Hülse 13 kann also beispielsweise ein Bestandteil des ersten Elements 5 bzw. des ersten Konnektors 11 sein oder des zweiten Elements 6 bzw. des zweiten Konnektors 12.

Die Hülse 12 kann beispielsweise (verliersicher) mit dem ersten Element 5 oder dem ersten Konnektor 12 (alternativ mit dem zweiten Element 6 bzw. dem zweiten Konnektor 12) verbunden sein. In diesem Fall kann die Hülse 13 in dem in Figur 1C gezeigten Zustand des Systems 1 (wenn die Konnektoren 11, 12 also voneinander getrennt sind) nicht von dem ersten Konnektor 11 über dessen vorderes Ende 15 hinweg abgestreift und so von dem ersten Konnektor 11 getrennt werden. Alternativ kann aber auch vorgesehen sein, dass die Hülse 13 von dem ersten Konnektor 12 über dessen vorderes Ende 15 hinweg abgestreift und von dem Konnektor 11 getrennt werden kann. Dann kann die Hülse 13 typischerweise auch wieder (wenn die Konnektoren 11, 12 voneinander getrennt sind) über das vordere Ende 15 des ersten Konnektors 11 (alternativ über das vordere Ende 16 des zweiten Konnektors 12) wieder über diesen aufgeschoben werden, um den in Figur 1C gezeigten Zustand wieder herzustellen. Das System 1 umfasst außerdem eine Rastvorrichtung mit mindestens einem Rastelement (in Figuren 1A bis 1C nicht gezeigt). Die Rastvorrichtung bzw. das mindestens eine Rastelement ist dazu ausgestaltet, zwischen der Sicherungshülse 13 und dem ersten Konnektor 11 und/oder zwischen der Sicherungshülse 13 und dem zweiten Konnektor 12 eine Rastverbindung herzustellen, wenn sich die Sicherungshülse relativ zum ersten Konnektor 11 und relativ zum zweiten Konnektor 12 in der Sicherungsposition befindet oder sich relativ zum ersten Konnektor 11 in der Halteposition befindet.

In den nachfolgenden Figuren sind fünf verschiedene Ausführungsbeispiele für das Systems 1 bzw. für Komponenten des Systems 1 gezeigt.

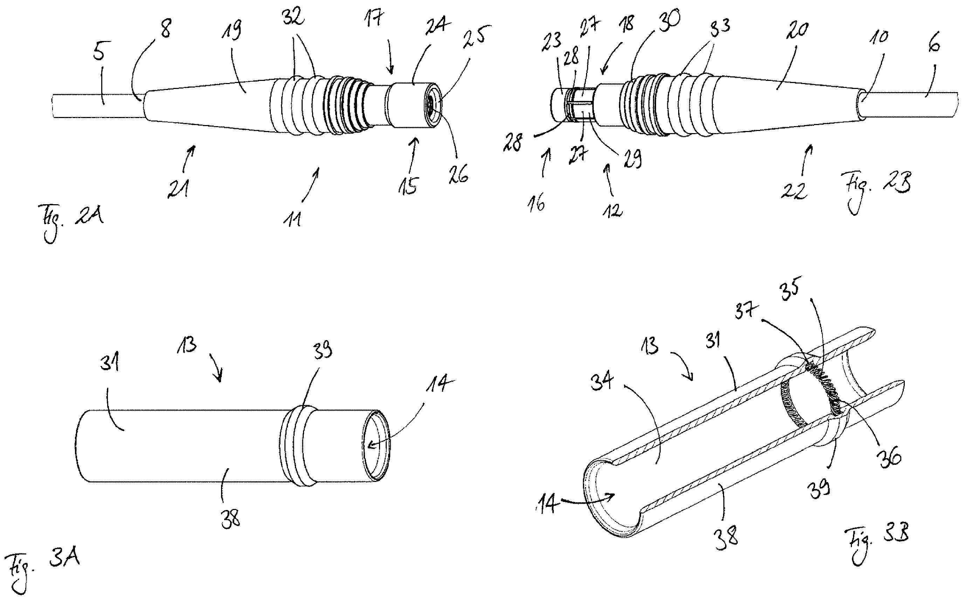

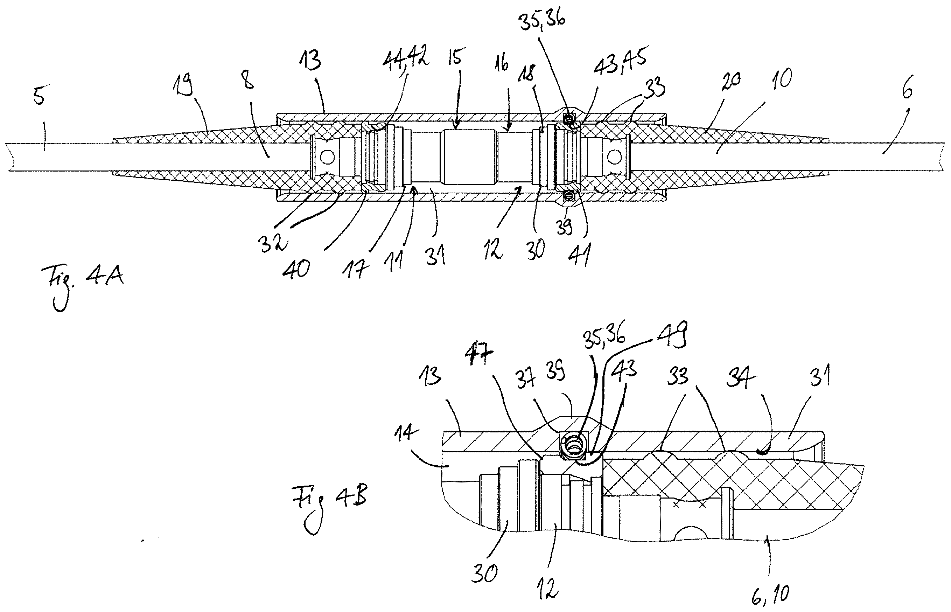

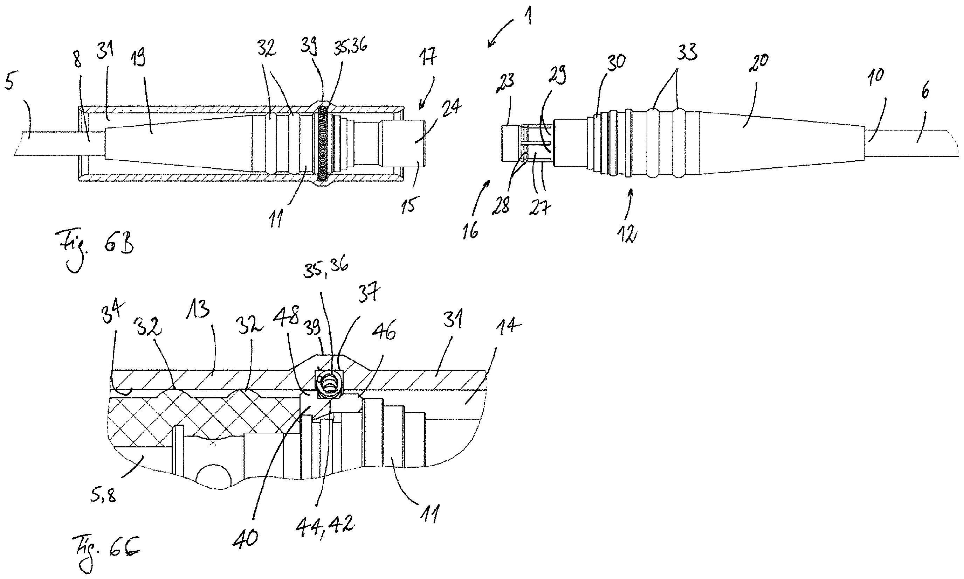

Figuren 2A bis 6B zeigen das System 1 bzw. einzelne Komponenten des Systems 1 aus Figuren 1A bis 1C gemäß Beispiel 1. Die Figuren 4A und 4B zeigen das System 1 gemäß Beispiel 1 in einer Konfiguration, in der die Konnektoren 11, 12 verbunden sind und sich die Sicherungshülse 13 in der Sicherungsposition befindet. Die Figur 5 zeigt das System 1 gemäß Beispiel 1 in einer Konfiguration, in der die Konnektoren 11, 12 verbunden sind und sich die Sicherungshülse 13 in der Halteposition befindet. Die Figuren 6A bis 6C zeigen den Konnektor 11 des Systems 1 bzw. das System 1 gemäß Beispiel 1 jeweils in einer Konfiguration, in der die Konnektoren 11, 12 getrennt sind und sich die Sicherungshülse 13 in der Halteposition befindet.

In dem in Figuren 2A bis 6B gezeigten Beispiel 1 des Systems 1 umfassen der erste Konnektor 11 und der zweite Konnektor 11 jeweils ein Gehäuse 17, 18, das aus einem metallischen Werkstoff wie beispielsweise Titan oder Edelstahl gebildet ist. Beide Konnektoren weisen außerdem einen hülsenförmigen Knickschutz 19, 20 auf, durch den das jeweilige zweite Ende 7,9 des ersten bzw. zweiten Elements 5, 6 hindurch verläuft, um ein Knicken möglichst zu vermeiden. Der Knickschutz 19, 20 ist am hinteren Ende 21, 22 des jeweiligen Konnektors 11, 12 fest mit dem Gehäuse 17, 18 des jeweiligen Konnektors 11, 12 verbunden.

Der erste Konnektor 11 ist als eine Buchse ausgestaltet und der zweite Konnektor 12 als ein Stecker. Der als Stecker ausgestaltete zweite Konnektor 12 weist an seinem vorderen Ende 16 ein Kupplungselement 23 auf, das hülsenförmig ausgestaltet ist. Der als Buchse ausgestaltete erste Konnektor 11 weist an seinem vorderen Ende 15 ein ebenfalls hülsenförmiges Aufnahmeteil 24 für das Kupplungselement 23 des Steckers auf. Sind die Konnektoren 11, 12 miteinander verbunden, wie in Figur 4A gezeigt ist, ist das Kupplungselement 23 in einem durch das Aufnahmeteil 24 definierten Aufnahmebereich 25 aufgenommen.

Die Konnektoren 11, 12 weisen korrespondierende Kontaktelemente 26 auf, die zur Übertragung elektrischer und/oder optischer Signale ausgestaltet und die an Oberflächen des Kupplungselements 23 bzw. des Aufnahmeteils 24 derart angeordnet sind, dass sie miteinander in Kontakt sind, wenn die beiden Konnektoren 11, 12 miteinander verbunden sind. Jedes der Kontaktelemente 26 ist mit einem der Leiter (nicht gezeigt) des jeweiligen Kabels verbunden.

Die lösbare Verbindung zwischen den beiden Konnektoren 11, 12 lässt sich praktisch beliebig oft herstellen und wieder lösen. Die beiden Konnektoren 11, 12 sind mit einem selbstverriegelnden Push-Pull-Mechanismus ausgestattet. Im gezeigten Beispiel etwa weist der zweite Konnektor 12 an seinem vorderen Ende 16 am Kupplungselement 23 mehrere metallische Riegelelemente 27 auf, die radial nach außen weisende Rastelemente 28 aufweisen. Der erste Konnektor 11 weist mehrere mit den Riegelelementen 27 korrespondierende Gegenriegelelemente (nicht dargestellt) auf, die auf einer Innenfläche des metallischen Aufnahmeteils 24 beispielsweise als vertiefte Aufnahmebereiche ausgestaltet sind. Die Riegelelemente 27 sind beweglich mit dem Gehäuse 18 des zweiten Konnektors 12 über Gelenk3 28 verbunden, die durch flexible elastische Bereich3 des Gehäuses 18 gebildet sind. Die Riegelelemente 27 sind zwischen einer vordefinierten Verriegelungsstellung und einer vordefinierten Entriegelungsstellung bewegbar. Die Verbindung zwischen den beiden Konnektoren 11, 12 ist nur lösbar ist, wenn sich die Riegelelemente in der Entriegelungsstellung befinden oder sich in diese bewegen können.

Der zweite Konnektor 12 weist ein mit den Riegelelementen 27 verbundenes bewegliches Betätigungselement 30 auf. Durch manuelle Betätigung (zum Beispiel durch axiales Ziehen des Betätigungselements 30 hin zum hinteren Ende 22 des zweiten Konnektors 12) können die Riegelelement 27 von der Verriegelungsstellung in die Entriegelungsstellung bewegt werden. Wenn das Betätigungselement nicht betätigt wird, befinden sich die Riegelelemente 27 (aufgrund der elastischen Gelenke 29) in der Verriegelungsstellung oder bewegen sich selbsttätig in diese hinein. Werden die Konnektoren 11, 12 also (mit korrekter Drehausrichtung gemäß Kontaktelemente 26 gemäß den in Figur 5 dargestellten Markierungen 50, 51) axial mit ihren vorderen Enden 15 und 16 ineinander geschoben (Stecker in Buchse), so rasten die Riegelelemente 27 selbsttätig in die jeweiligen Aufnahmen ein und die axial stabile, lösbare Verbindung zwischen den beiden Konnektoren 11, 12 ist hergestellt und automatisch verriegelt.

Die in Figuren 3A und 3B gezeigte Sicherungshülse 13 weist einen hülsenförmigen Hauptkörper 31 auf, der den oben beschriebenen Hohlraum 14 bildet, um in der Sicherungsposition die beiden vorderen Enden 15, 16 der Konnektoren 11, 12 einschließlich der Riegelelemente 27, Gegenriegelemente und des Betätigungselements 30 in sich aufzunehmen und zu überdecken. Dadurch, dass das Betätigungselement 30 nicht zugänglich und somit auch nicht (unbeabsichtigt) manuell betätigt werden kann, solange sich die Sicherungshülse 13 in der Sicherungsposition befindet, ist die Verbindung zwischen den Konnektoren vor einem (ungewollten) Entriegeln und Lösen geschützt. Dadurch, dass die Hülse 13 in der Sicherungsposition eingerastet ist, wird ein ungewolltes Bewegen der Hülse aus der Sicherungsposition und ein darauf prinzipiell ermöglichtes ungewolltes Entriegeln der Verbindung der Konnektoren verhindert oder zumindest erschwert.

Die Konnektoren 11,12 und die Sicherungshülse 13 der nachfolgenden Beispiele 2 bis 5 entsprechen den in Figuren 2A, 2B gezeigten Konnektoren 11, 12 und der in Fign. 3A und 3B gezeigten Sicherungshülse 13 des Beispiels 1 zumindest in den oben beschriebenen Merkmalen. Unterschiede zwischen den Beispielen 1 bis 5 ergeben sich durch unterschiedliche Ausgestaltungen der Rastvorrichtung, der Rastelemente und Rastgegenelementen, sowie ggf. durch weitere Elemente, wie nachfolgend beschrieben.

Um Sicherungshülse 13 des in Figuren 2A bis 6B gezeigten Beispiels 1 in radialer Richtung zu stützen und zu führen, weisen die Konnektoren 11, 12 auf ihren Außenflächen beispielsweise Stützflächen 32, 33 auf, die beispielsweise als ringförmige Stege ausgestaltet sind und/oder vorzugsweise wie Dichtflächen eine innere Oberfläche 34 der Hülse (ringsum) kontaktieren, siehe Figuren 4A bis 6B.

Die Rastvorrichtung des in Figuren 2A bis 6B gezeigten Beispiels 1 umfasst als ein Rastelement 35 ein in sich geschlossenes elastisches Ringelement 36. Im gezeigten ersten Beispiel handelt es sich hierbei um eine ringförmig gewickelte Spiralfeder. Es könnte alternativ aber auch als ein Axialsicherungsring oder ein O-Ring ausgestaltet sein. Wie in Figur 3B gezeigt ist, weist die innere Oberfläche 34 der Hülse 13 eine Ringnut 37 auf, in der das Ringelement 36 durch Eigenspannung dauerhaft befestigt ist. Korrespondierend zur Ringnut 37 weist die Hülse 13 auf ihrer äußeren Oberfläche 39 einen ringförmigen Steg als strukturierte Grifffläche 39 der Hülse 13 auf.

Die Rastvorrichtung umfasst außerdem Gegenrastelemente 40, 41, bei denen es sich um ringförmige Elemente handelt, die Bereiche einer äußeren Oberflächen 42, 43 des ersten bzw. zweiten Konnektors 11, 12 bilden. Das Rastelement 35 ist ausgestaltet, in einer Ringnut 44 in der äußeren Oberfläche 42 des ersten Konnektors 11 einzurasten, wenn sich die Sicherungshülse in der Halteposition befindet, siehe Figuren 5 und 6A bis 6C. Das Rastelement 35, 36 ist außerdem ausgestaltet, in einer Ringnut 45 auf der äußeren Oberfläche 43 des Gegenrastelements 41 des zweiten Konnektors 12 einzurasten, wenn sich die Sicherungshülse 13 in der Sicherungsposition befindet, siehe Figuren 4A und 4B. Eine umgekehrte Konfiguration wäre natürlich ebenso möglich (Gegenrastelemente 40, 41 mit Hülse 13 verbunden, Rastelement 35 mit erstem oder zweiten Konnektor 11, 12 fest verbunden).

Das Rastelement 35 und die Gegenrastelemente 40 und 41 sind derart ausgestaltet, dass die Sicherungshülse 13 durch manuelles axiales Verschieben aus der Sicherungsposition wie auch aus der Halteposition heraus bewegbar ist, wenn eine auf die Sicherungshülse 13 einwirkende axiale Verschiebekraft einen vordefinierten Schwellwert überschreitet. Insbesondere bilden die Gegenrastelemente Steigflächen 46, 47 aus, die ein Gleiten des Rastelements 35 in die jeweilige Ringnut 44 bzw. 45 erleichtern, sowie jeweils einen (endständigen) Anschlag 48,49, der ein ungewolltes axiales Verschieben der Hülse 13 über die Sicherungsposition bzw. Halteposition hinweg verhindert, so dass die Hülse 13 nur zwischen der Sicherungsposition und der Halteposition bewegt werden kann, wenn die Konnektoren 11, 12 miteinander verbunden sind.

Wie in Figur 5 gezeigt ist, sind auf den vorderen Enden 15, 16 des ersten und zweiten Konnektors 11, 12 jeweils eine (pfeilförmige) Markierung 50, 51 angeordnet, mit der eine korrekte Drehausrichtung der Konnektoren zueinander überprüft werden kann. Vorteilhafterweise sind die Markierungen 50, 51 für den Nutzer gut sichtbar, wenn sich die Hülse 13, wie in Figur 5A gezeigt, in der Halteposition befindet.

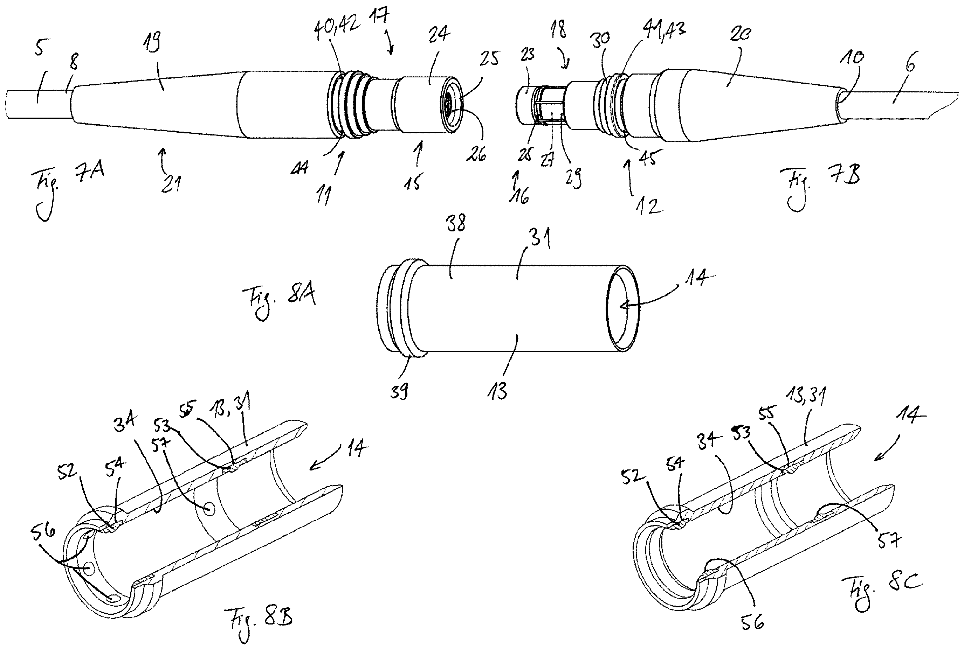

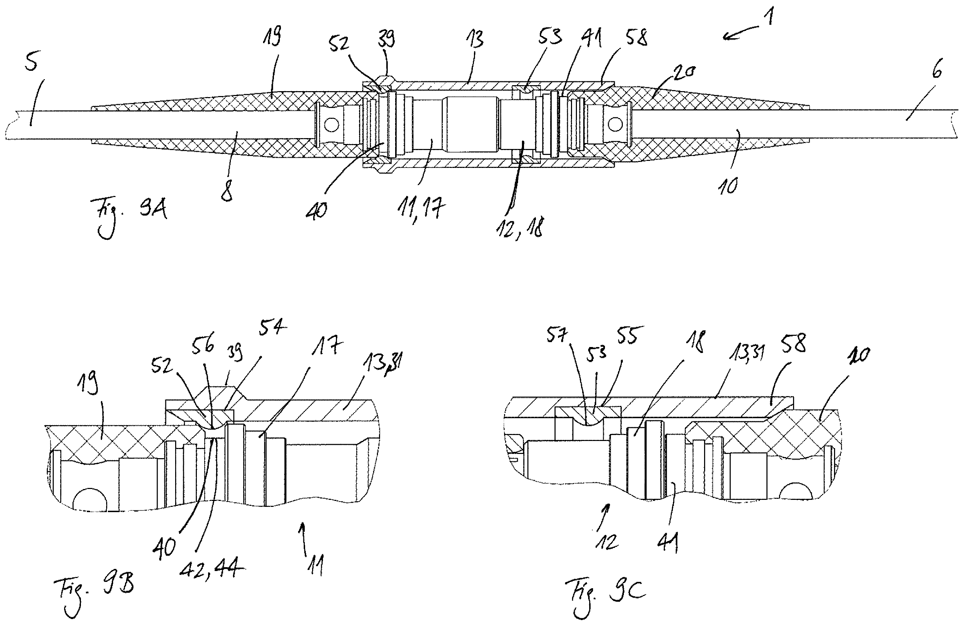

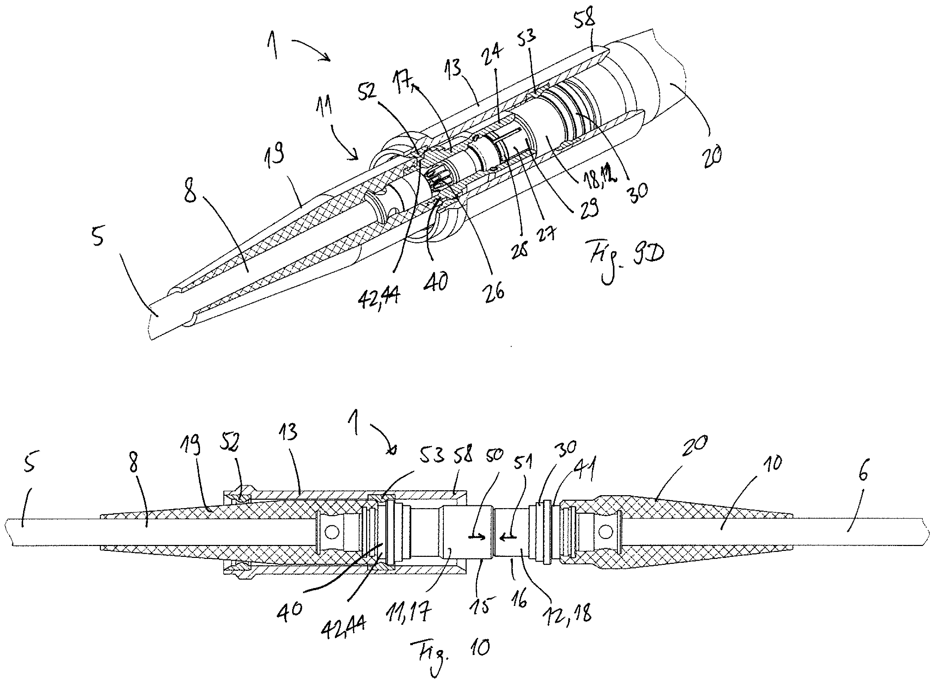

Figuren 7A bis 10 zeigen das System 1 aus Figuren 1A bis 1C bzw. einzelne Komponenten hiervon gemäß dem Beispiel 2. Die Figuren 9A bis 9D zeigen das System 1 gemäß Beispiel 2 in einer Konfiguration, in der die Konnektoren 11, 12 verbunden sind und sich die Sicherungshülse 13 in der Sicherungsposition befindet. Die Figur 10 zeigt das System 1 gemäß Beispiel 1 in einer Konfiguration, in der die Konnektoren 11, 12 verbunden sind und sich die Sicherungshülse 13 in der Halteposition befindet.

Im Unterschied zum Beispiel 1 weist die Rastvorrichtung des Beispiels 2 mehrere Rastelemente auf, nämlich ein erstes Rastelement 52 und ein zweites Rastelement 53. Diese sind ringförmig ausgestaltet und aus einem elastischen ersten Kunststoff gefertigt. In der in Figur 8B gezeigten ersten Variante der Hülse 13 wie auch in der in Figur 8C gezeigten zweiten Variante der Hülse 13 weist die Hülse 13 einen aus einem relativ starren zweiten Kunststoff gefertigten Hauptkörper 31 auf, auf dessen inneren Oberfläche 34 jeweils eine ringförmige Vertiefung 54, 55 angeordnet ist, die das erste Rastelemente 52 bzw. das zweite Rastelement 53 teilweise in sich aufnimmt. Die Rastelemente 52, 53 weisen radial nach innen ragende Ausbuchtungen 56, 57 auf. Im Fall der in Figur 8B gezeigten ersten Variante haben die Ausbuchtungen 56, 57 jeweils die Form mehrerer azimutal voneinander beabstandeter Noppen. Im Fall der in Figur 8C gezeigten zweiten Variante haben die Ausbuchtungen 56, 57 jeweils die Form eines ringförmigen Stegs. Bei der Hülse 13 gemäß der ersten oder zweiten Variante handelt es sich beispielsweise um ein 2K-Spritzgusteil, bei dem der Hauptkörper 31 und die Rastelemente 52, 53 in einem 2K-Spritzgussverfahren mit dem oben genannten ersten und zweiten Kunststoff hergestellt worden sind.

Die Konnektoren 11, 12 des Beispiels 2 unterscheiden sich von den Konnektoren 11, 12 des Beispiels 1 im Wesentlichen nur durch das zu den Rastelementen 52, 53 korrespondierende Gegenrastelement 40, das im Beispiel 2 durch eine Bereich auf der Oberfläche 42 des Gehäuses 17 des ersten Konnektors 11 gebildet wird. In diesem Bereich der Oberfläche 42 weist das Gehäuse eine Ringnut 44 auf, in die das erste Rastelement 52 mit den noppenförmigen Ausbuchtungen 56 (Variante 1) bzw. der ringförmigen Ausbuchtung 56 (Variante 2) einrastet, wenn sich die Hülse 13 in der Sicherungsposition befindet, siehe Figuren 9A bis 9C, und in die das zweite Rastelement 52 mit den noppenförmigen Ausbuchtungen 56 (Variante 1) bzw. der ringförmigen Ausbuchtung 56 (Variante 2) einrastet, wenn sich die Hülse 13 in der Halteposition, siehe Figur 10.

Wie in Figur 9A und 9C deutlich zu erkennen ist, ist der Außendurchmesser des Knickschutzes 20 des zweiten Konnektors 12 derart bemessen, dass die Sicherungshülse 13 in der Sicherungsposition den Knickschutz 20 mit einem vorderen Ende 58 der Hülse von außen berührt und auf diese Weise durch den Knickschutz 20 radial abgestützt und stabilisiert wird. Wie in Figur 9A und 9C deutlich zu erkennen ist, ist der Außendurchmesser des Knickschutzes 19 des ersten Konnektors 11 nur geringfügig kleiner als ein Innendurchmesser der inneren Oberfläche 34 der Hülse 13, so dass die Sicherungshülse 13 in der Halteposition durch den Knickschutz 19 radial abgestützt und stabilisiert wird.

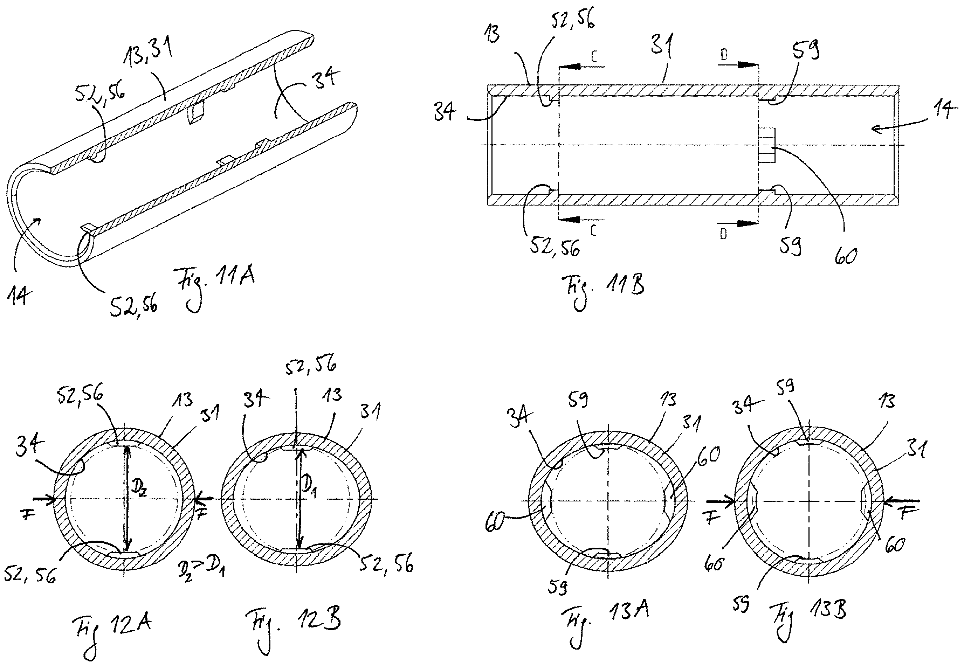

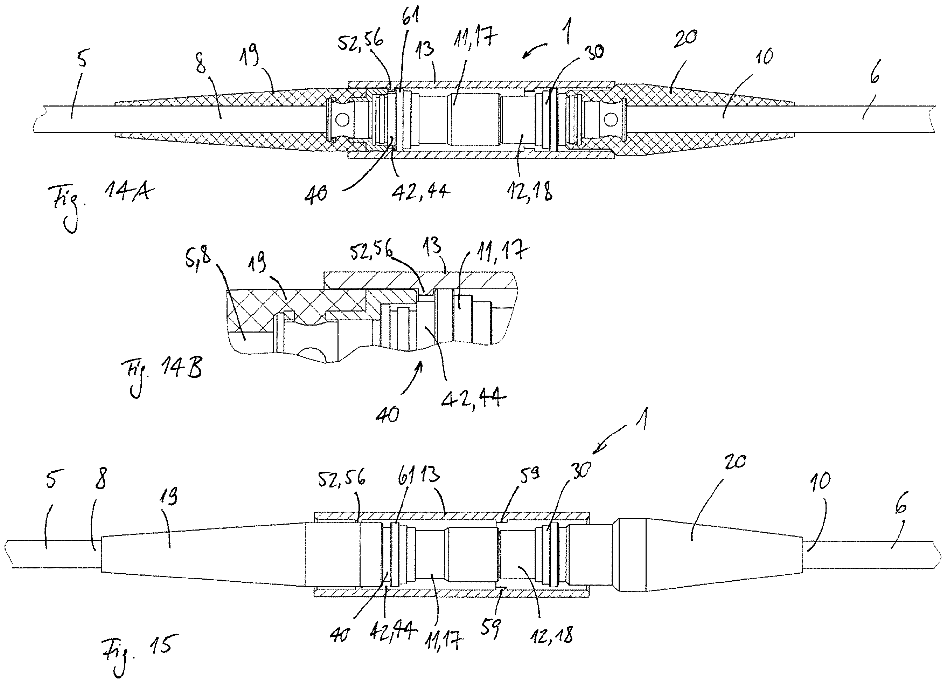

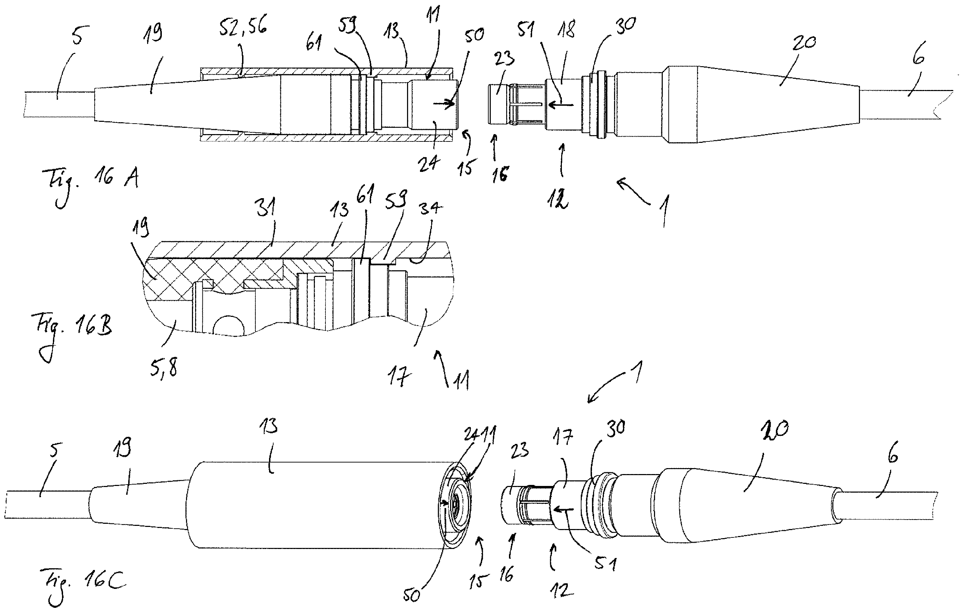

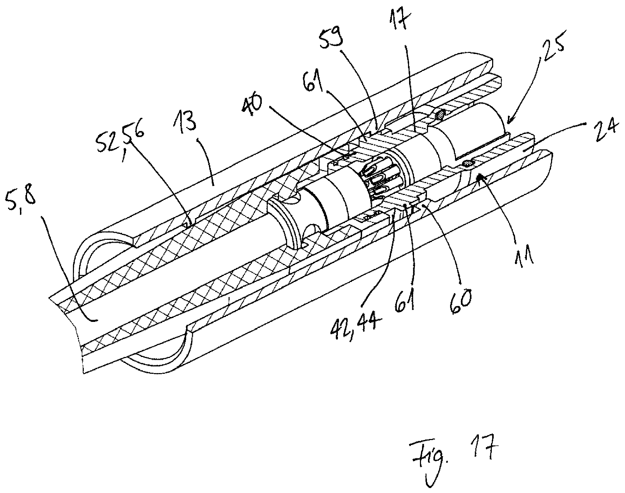

Figuren 11A bis 17 zeigen das System 1 aus Figuren 1A bis 1C bzw. einzelne Komponenten hiervon gemäß dem Beispiel 3. Die Figuren 14A und 14B zeigen das System 1 in einer Konfiguration, in der die Konnektoren 11,12 verbunden sind und sich die Sicherungshülse 13 in der Sicherungsposition befindet. Die Figur 15 zeigt System 1 in einer Konfiguration, in der die Konnektoren 11, 12 verbunden sind und sich die Sicherungshülse 13 zwischen der Sicherungsposition und der Halteposition befindet. Die Figuren 16A bis 17 zeigen das System 1 in einer Konfiguration, in der die Konnektoren 11, 12 getrennt sind und sich die Sicherungshülse 13 in der Halteposition befindet.

Ähnlich dem Beispiel 2 umfasst die Rastvorrichtung im Beispiel 3 Rastelemente 52, die in Form von Ausbuchtungen 56 von einer inneren Oberfläche 34 der Hülse 13 radial nach innen vorspringen. Die Ausbuchtungen 56 sind als Stege ausgestaltet. Siehe Figuren 11A und 11B.

Figur 12B zeigt die Sicherungshülse 13 in einem entspannten Ausgangszustand, den sie dann einnimmt, wenn keine äußere Kraft, insbesondere keine radiale Kompressionskraft, auf sie einwirkt. Die beiden Ausbuchtungen 56 nehmen in dem Ausgangszustand der Sicherungshülse 13 einen ersten Abstand D1 voneinander ein. Der erste radiale Abstand D1 ist in einer ersten radialen Richtung definiert und derart bemessen, dass die beiden Ausbuchtungen 56 in der Oberfläche 42 des ersten Konnektors 12, die in diesem Beispiel durch ein mit einer Ringnut 44 versehenen Gegenrastelement 40 gebildet wird, einrasten oder eingerastet sind, wenn sich die Sicherungshülse 13 im Ausgangszustand und relativ zu dem ersten Konnektor 11 und dem mit dem ersten Konnektor 11 verbundenen zweiten Konnektor 12 in der Sicherungsposition befindet. Wie in Figuren 14A und 14B gezeigt ist, tauchen die beiden Ausbuchtungen 56 dann in diese Ringnut 44 ein.

Wie in Figur 12A gezeigt ist, ist die Sicherungshülse 13 durch eine in einer zweiten radialen Richtung, die von der ersten radialen Richtung verschieden ist, auf die Sicherungshülse 13 einwirkende radiale Kompressionskraft F in einen gespannten Zwischenzustand verformbar. Diese Kompressionskraft kann durch manuelles Zusammendrücken bewirkt werden. Die beiden Ausbuchtungen 56 nehmen in dem Zwischenzustand der Sicherungshülse 13 einen in der ersten radialen Richtung definierten zweiten Abstand D2 voneinander ein. Der zweite Abstand D2 ist größer als der erste Abstand und derart bemessen, dass die beiden Ausbuchtungen nicht (mehr) in der Oberfläche 40 des ersten Konnektors 11 eingerastet sind (sie tauchen dann also nicht in die Ringnut 44 ein), wenn sich die Sicherungshülse 13 in ihrem Zwischenzustand und sich gleichzeitig in der Sicherungsposition befindet. Zu diesem Zweck ist D2 (zumindest geringfügig) größer als ein größter Außendurchmesser des Gehäuses 17 des ersten Konnektors 11. Daher kann die Hülse 13 in diesem Zwischenzustand durch axiales Verschieben relativ zum ersten und zweiten Konnektor 11, 12 in die Sicherungsposition hinein oder wieder aus dieser heraus bewegt werden, wie in Figur 15 gezeigt ist. Die genannte Verformung der Hülse 13 ist elastisch, so dass sich die Sicherungshülse 13 von selbst wieder in den in Figur 12B gezeigten Ausgangszustand zurück verformt, sobald die radiale Kompressionskraft F nicht mehr auf sie einwirkt.

Die Hülse 13 weist auf ihrer Innenfläche 34 außerdem erste Ausbuchtungen 59 und zweite Ausbuchtungen 60 auf, welche im Zusammenspiel mit einem Anschlag 61, der an dem ersten Konnektor 11 angeordnet ist, bewirken, dass die Hülse 13 ausgehend von der Sicherungsposition bis zu der durch diesen Anschlag 61 definierten Halteposition verschoben werden kann, wie in Figuren 16A und 16B gezeigt ist, aber nicht darüber hinaus, und zwar unabhängig davon, ob sich die Hülse im Ausgangszustand oder im Zwischenzustand befindet. Zu diesem Zweck sind die ersten Ausbuchtungen 59 beispielsweise derart angeordnet und bemessen, dass ihr Abstand in der ersten Richtung kleiner als der Außendurchmesser des Anschlags 61 in der ersten Richtung ist, wenn sich die Sicherungshülse 13 im Ausgangszustand befindet, wie in Figur 13A gezeigt ist. Außerdem sind die zweiten Ausbuchtungen 60 derart angeordnet und bemessen, dass ihr Abstand in der zweiten Richtung kleiner als der Außendurchmesser des Anschlags 61 in der zweiten Richtung ist, wenn sich die Sicherungshülse 13 im Zwischenzustand befindet, wie in Figur 13B gezeigt. Wie in Figuren 12B und 13B gezeigt ist, weist der Hauptkörper der Hülse 13 einen Querschnitt auf, der im Ausgangszustand eine ovale Form und, wie in Figuren 12A und 13B gezeigt ist, im Zwischenzustand eine kreisrunde Form hat. Der Anschlag 61 hat beispielsweise eine kreisförmige Außenkontur mit einem Au-ßendurchmesser, der etwa D2 entspricht oder geringfügig kleiner ist.

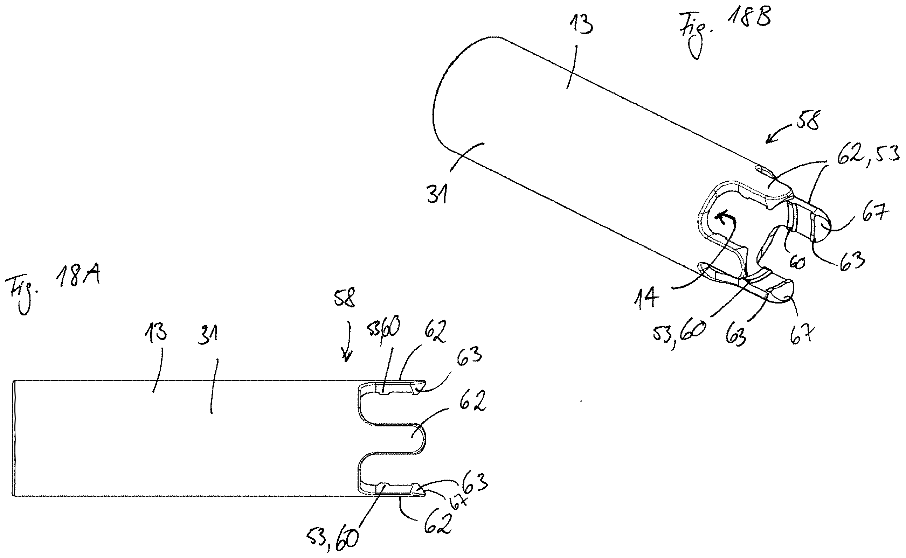

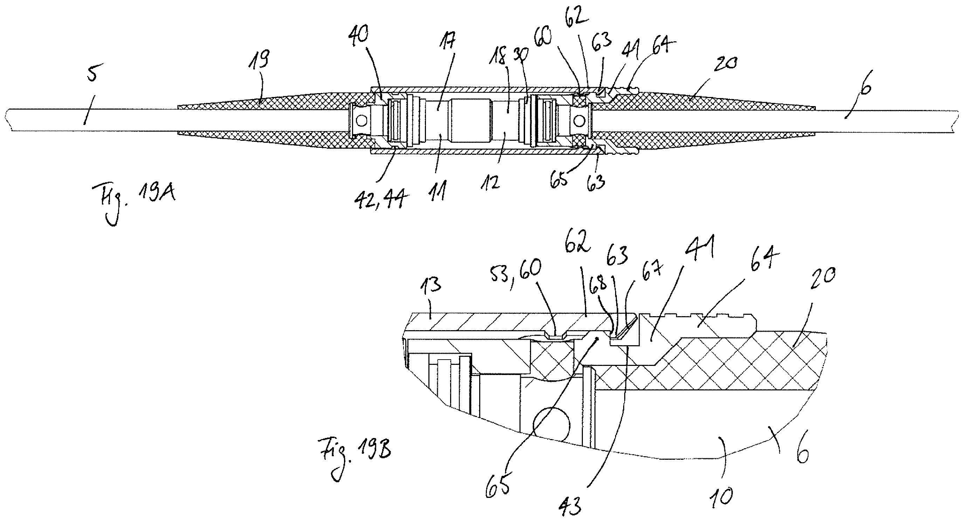

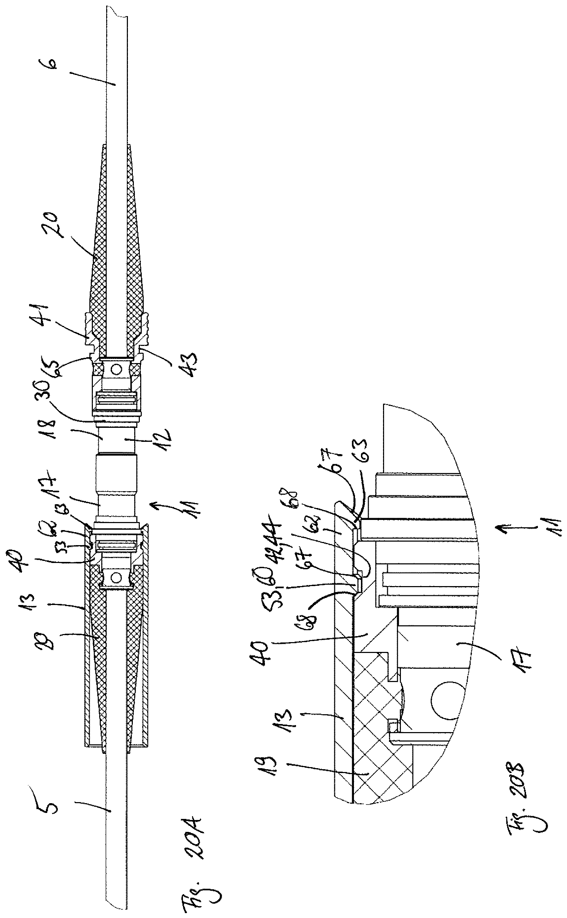

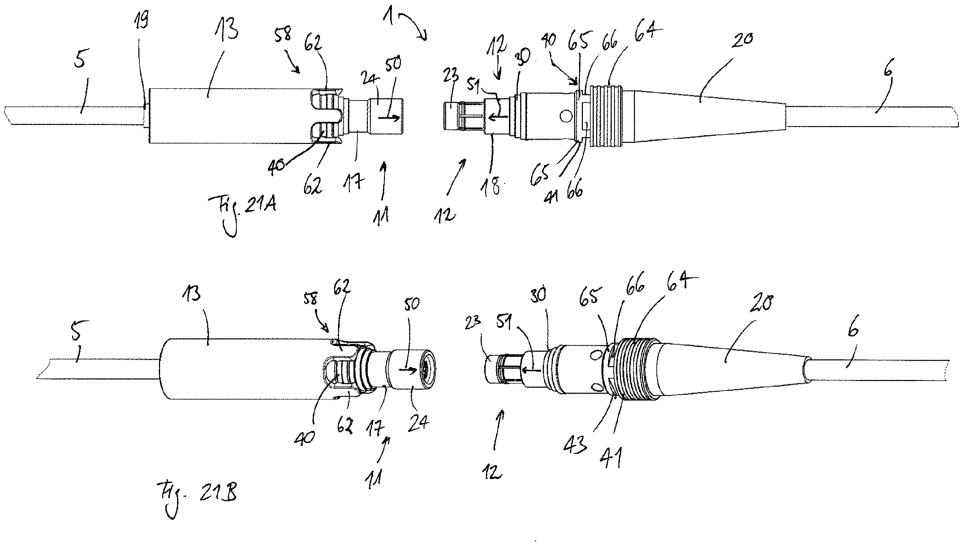

Figuren 18A bis 21B zeigen das System 1 aus Figuren 1A bis 1C bzw. einzelne Komponenten hiervon gemäß dem Beispiel 4. Die Figuren 19A und 19B zeigen das System 1 gemäß Beispiel 4 in einer Konfiguration, in der die Konnektoren 11, 12 verbunden sind und sich die Sicherungshülse 13 in der Sicherungsposition befindet. Figuren 20A und 20B zeigen das System 1 in einer Konfiguration, in der die Konnektoren 11, 12 verbunden sind und sich die Sicherungshülse 13 in der Halteposition befindet. Die Figuren 21A und 21B zeigen das System 1 in einer Konfiguration, in der die Konnektoren 11, 12 getrennt sind und sich die Sicherungshülse 13 in der Halteposition befindet.

Das Beispiel 4 unterscheidet sich von den Beispielen 1 bis 3 dadurch, dass die Sicherungshülse 13 des Systems 1 an ihrem vorderen Ende 58 mehrere sich in axialer Richtung erstreckende Rastarme 61 aufweist, welche jeweils ein Rastelement 52 der Rastvorrichtung in Form eines radial nach innen vorspringenden Rastzahns 63 aufweist. Die Rastarme 61 weisen außerdem jeweils ein weiteres Rastelement 53 in Form einer radial nach innen weisenden Ausbuchtung 60 auf.

Ein Bereich der Oberfläche 42 des zweiten Konnektors 12 wird durch ein beispielsweise aus einem Kunststoff gebildetes, ringförmiges Griffteil 64 gebildet, das außerdem ein Gegenrastelement 41 für die Rastzähne 63 bildet. Das Gegenrastelement 41 weist eine Stufe 65 auf. Befindet sich die Hülse 13 in der Sicherungsposition, wie in Figuren 19A und 19B gezeigt, hintergreifen die Rastarme 62 mit ihren Rastzähnen 63 die Stufe 65. Wie aus Figur 21B zu erkennen ist, weist das Gegenrastelement 41 Entriegelungssteigflächen 66 auf, die rückseitig an die Stufe 65 angrenzen. Die Rastarme 62 gleiten bei einer (azimutalen) Drehung der Hülse 13 in der Sicherungsposition über die Entriegelungssteigflächen 66 und werden dabei radial auseinandergespreizt, wobei die Rastzähne 63 der Rastarme 62 die Entriegelungssteigflächen 66 kontaktieren.

Wie in Figur 19B zu erkennen ist, weisen die Rastarme 62 auf der Vorderseite des jeweiligen Rastzahns 63 eine Steigfläche 67 und auf der Rückseite des jeweiligen Rastzahns 63 eine Haltefläche 68 auf.

Während die Halteflächen 68 im Wesentlichen senkrecht zur Längsachse der Hülse 13 ausgerichtet sind, sind die Steigflächen 67 hierzu so angewinkelt, dass die Sicherungshülse 13 durch axiales Verschieben der Sicherungshülse 13 in die Sicherungsposition geschoben werden kann. Die Hülse 13 kann aber nur nach der oben beschriebenen Dreh- und Gleitbewegung über die Entriegelungssteigflächen aus der Sicherungsposition wieder heraus bewegt werden.

Die weiteren Rastelemente 53 weisen auf den Vorder- bzw. Rückseiten der jeweiligen Ausbuchtungen 60 ebenfalls Steigflächen 67 und Halteflächen 68 auf, die alle derart angewinkelt sind, dass die Sicherungshülse 13 durch axiales Verschieben in die Halteposition hinein und wieder aus ihr heraus bewegt werden kann, sofern eine definierte Verschiebekraft aufgewendet wird. Die Rastelemente 53 befinden sich in der Halteposition mit einem korrespondierenden Gegenrastelement 40 des ersten Konnektors 11 in einem gegenseitigen Eingriff. Das Gegenrastelement 40 bildet einen Bereich der Oberfläche 42 des ersten Konnektors 11 und weist eine Ringnut 44 auf, in die die Ausbuchtungen 60 aufgenommenen sind, wenn sich die Hülse 13 in der Halteposition geschoben wird.

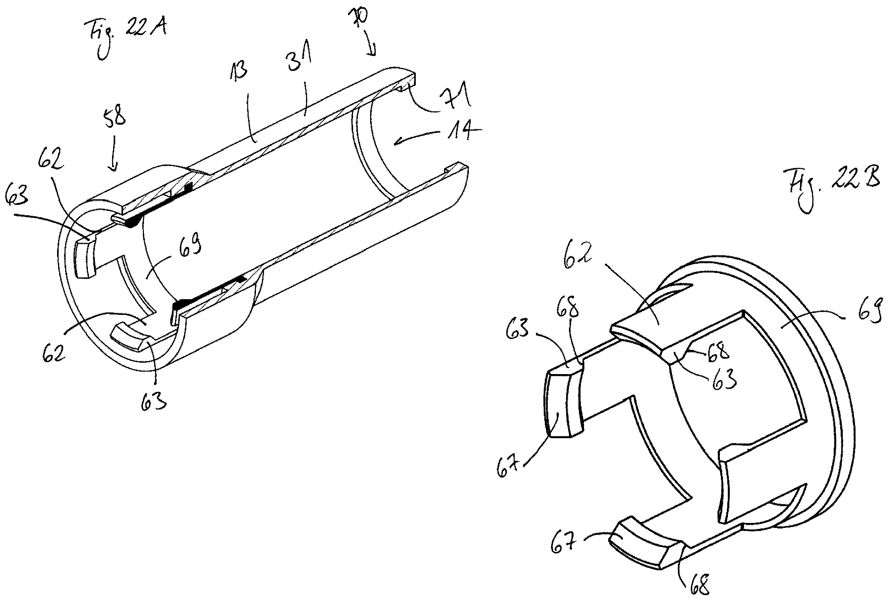

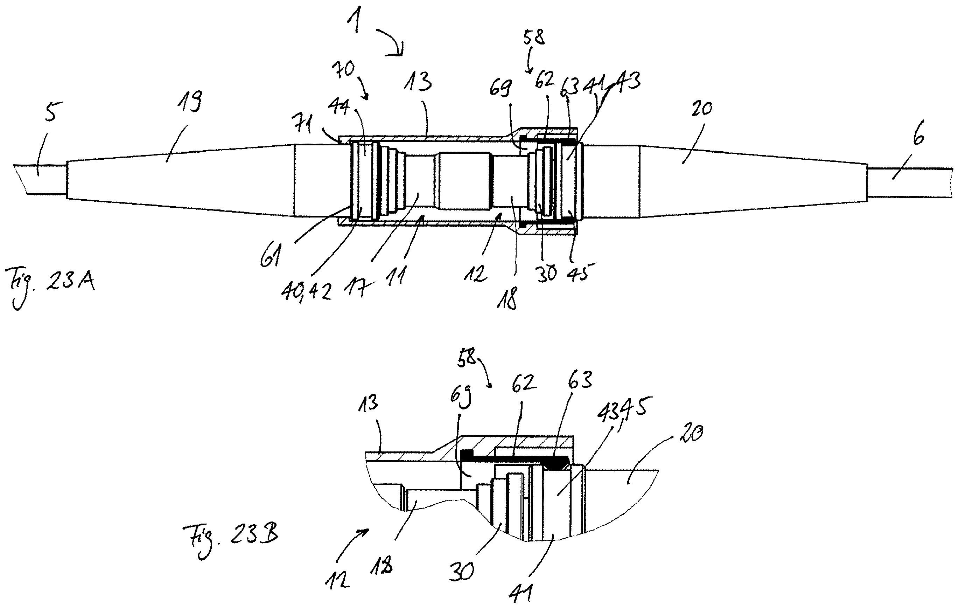

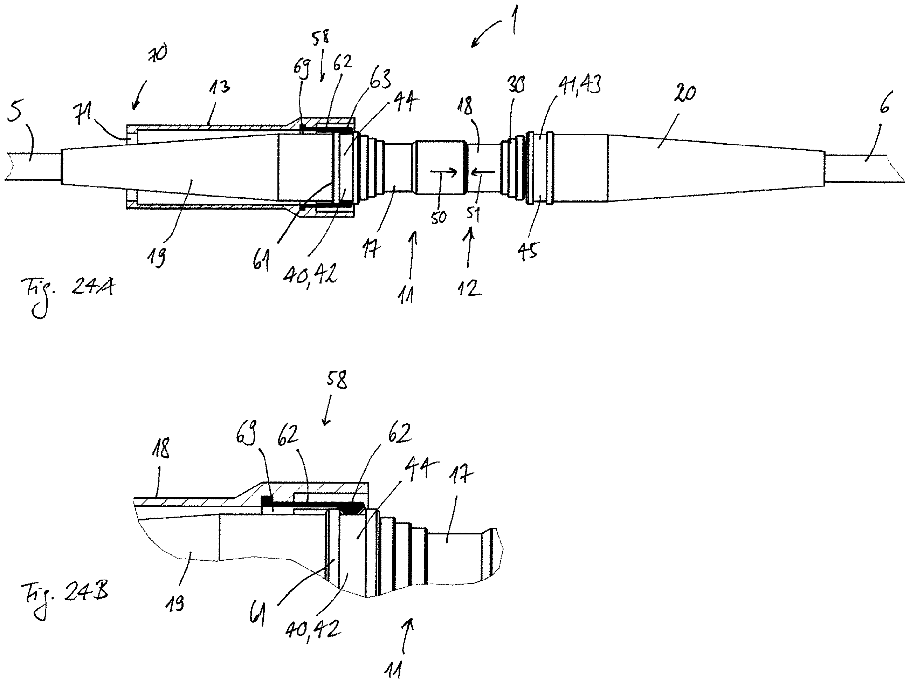

Figuren 22A bis 24A zeigen das System 1 aus Figuren 1A bis 1C bzw. einzelne Komponenten hiervon gemäß dem Beispiel 5. Die Figuren 23A und 23B zeigen das System 1 in einer Konfiguration, in der die Konnektoren 11, 12 verbunden sind und sich die Sicherungshülse 13 in der Sicherungsposition befindet. Figuren 24A und 24B zeigen das System 1 in einer Konfiguration, in der die Konnektoren 11, 12 verbunden sind und sich die Sicherungshülse 13 in der Halteposition befindet.

Im Beispiel 5 weist die Sicherungshülse 13 ebenso wie in Beispiel 4 an ihrem vorderen Ende 58 mehrere sich in axialer Richtung erstreckende Rastarme 62 auf, welche jeweils ein Rastelement der Rastvorrichtung in Form eines radial nach innen vorspringenden Rastzahns 63 aufweist. Während die Rastarme 62 im Beispiel 4 als axiale Verlängerungen des im Wesentlichen zylindrischen Hauptkörpers 31 der Hülse 13 ausgestaltet sind, weist die Rastvorrichtung im Beispiel 5 einen hülsenförmigen, beispielsweise aus einem metallischen Werkstoff gebildeten Träger 69 auf, siehe Figur 22B, von dem ausgehend sich die Rastarme 62 in axialer Richtung erstrecken. Der Träger 69 ist fest mit dem Hauptkörper 31 der Hülse 13 verbunden, beispielsweise durch Verkleben oder formschlüssige Fügung oder Verpressung. Der Träger ist ebenso wie die Rastarme 62 in dem durch den Hauptkörper 31 der Hülse 13 definierten Hohlraum 14 angeordnet.

Der zweite Konnektor 12 des Beispiels 5 unterscheidet sich von dem zweiten Konnektor 12 des Beispiels 4 darin, dass das Gegenrastelement 41 im Beispiel 5 keine Entriegelungssteigflächen 66 auf der Rückseite der Stufe 65 aufweist. Um eine Entriegelung ohne Drehen der Hülse im Beispiel 5 zu ermöglichen, sind statt dessen die rückseitigen (ebenen) Halteflächen 68 der Rastzähne 63 gegenüber einer Bezugsebene, die senkrecht zur Längsachse der Sicherungshülse 13 orientiert ist, angewinkelt. Vorzugsweise liegt der Winkel, der jeweils zwischen der Haltefläche 68 und der Bezugsebene eingeschlossen wird, zwischen 30° und 60°, vorliegend beispielsweise bei etwa 45°. Auf diese Weise ist die Kraft definiert, die erforderlich ist, die Hülse 13 aus der Sicherungsposition axial heraus zu schieben. Wie in Fig. 23B dargestellt ist, kann die Haltefläche der Ringnut 45 beispielsweise in einem Winkel von 90° zur Längsachse des zweiten Konnektors 12 orientiert sein. Es ist aber alternativ auch möglich, die Ringnut 45 der Oberfläche 43 des Rastelementes 41 zur angewinkelt zu gestalten. Entsprechendes gilt auch für die Haltefläche der Ringnut 44 der Oberfläche 42 des Rastelementes 40.

Wie in Figur 24A gezeigt ist, kann die Hülse 13 durch axiales Verschieben in die Halteposition geschoben werden, in der die Rastzähne 63 in der durch das Gegenrastelement 40 gebildeten Ringnut 44 auf der Oberfläche 42 des ersten Konnektors 11 einrasten. Beim Einrasten in der Halteposition können die Halteflächen 68 aufgrund ihrer angewinkelten Ausrichtung nun wie Steigflächen wirken. Auf diese Weise kann im Vergleich zum Beispiel 4 auf die zusätzlichen Rastelemente 53 an den Rastarmen 62 für das Einrasten der Hülse 13 in der Halteposition verzichtet werden.

Wie beispielsweise in Figuren 22A zu erkennen ist, weist die Hülse 13 am hinteren Ende 70 auf der inneren Oberfläche 34 eine radial nach innen gerichtete stegförmige Ausbuchtung 71 auf, die im Zusammenspiel mit einem Anschlag 61 an dem ersten Konnektor 11 ein vollständiges Abstreifen der Hülse 13 von dem ersten Konnektor 13 und somit ein Verlieren der Hülse 13 verhindert.

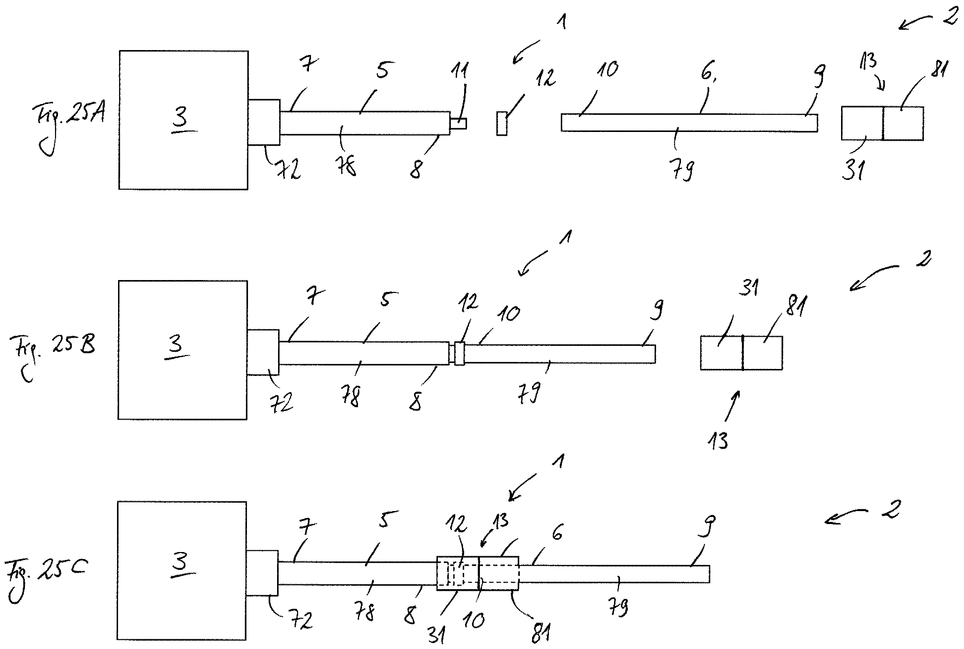

Figuren 25A bis 25C zeigen jeweils eine schematische Darstellung eines weiteren (zweiten) Systems 1 hier vorgeschlagener Art zum Sichern einer lösbaren Verbindung zwischen zwei als Hohlkörper 78, 79 ausgestalteten Elementen 5, 6. Dieses System 1 ist beispielsweise ein Bestandteil eines weiteren Blutpumpensystems 2 hier vorgeschlagener Art. Prinzipiell kann es sich bei den in Figuren 1A bis 1C und 25A bis 25C gezeigten Blutpumpensystemen 2 um ein und dasselbe Blutpumpensystem 2 handeln. In diesem Fall umfasst das Blutpumpensystem 2 also zwei Systeme 2 hier vorgeschlagener Art auf, wobei das erste System 1, wie oben beschrieben, die lösbare Verbindung zwischen zwei als Kabel ausgestalteten Elementen 5, 6 sichert, und das zweite System 2, wie nachfolgenden beschrieben, die lösbare Verbindung zwischen den beiden zwei als Hohlkörper 78, 79 ausgestalteten Elementen 5, 6 des Blutpumpensystems 2 sichert. Jedoch kann das Blutpumpensystem 2 auch nur das erste oder nur das zweite dieser beiden Systeme 2 umfassen.

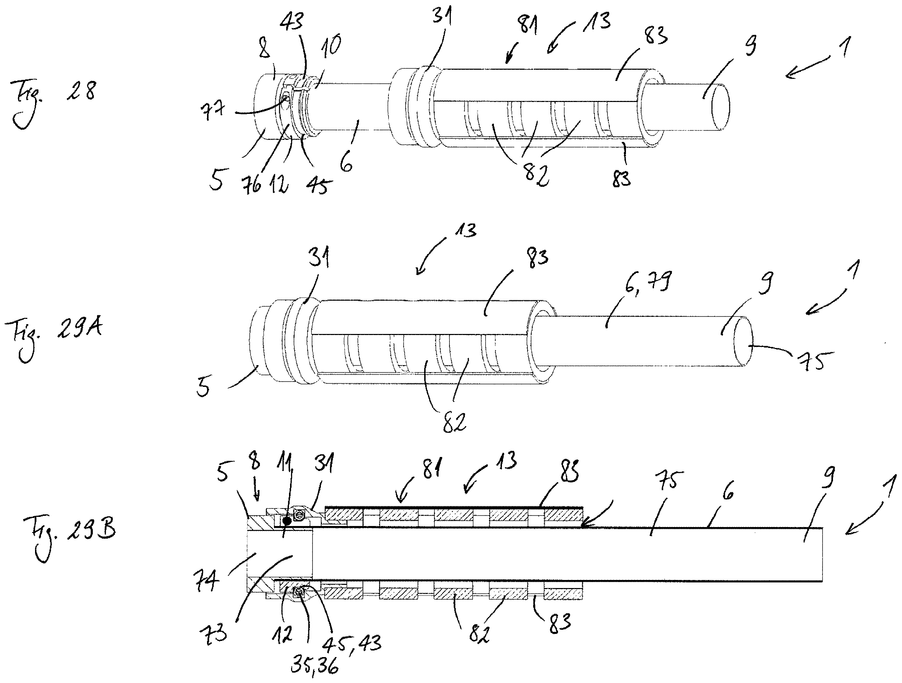

Das in Figuren 25A bis 25C gezeigte Blutpumpensystem 2 umfasst eine implantierbare Blutpumpe 3. Das erste Element 5 ist ein pumpenseitiger Hohlkörper 78 mit einem ersten Ende 7, das mit einem Pumpenauslass 72 der Blutpumpe 3 flüssigkeitsdicht verbindbar bzw. flüssigkeitsdicht verbunden ist, und mit einem zweiten Ende 8. Das zweite Element 6 in Form des weiteren Hohlkörpers 79 hat ein erstes Ende 9, das beispielsweise mit einem Blutgefäß oder Herzen eines Patienten verbindbar ist, und ein zweites Ende 10. Die beiden Elemente 5, 6 bilden somit jeweils einen Fließkanal 74 bzw. 75 zum Leiten von Blut. Siehe beispielsweise Figur 29B.

Bei dem Hohlkörper 78 des ersten Elements 5 handelt es sich beispielsweise um ein Schlauch oder ein Rohr. Der Hohlkörper 78 ist beispielsweise aus Silikon oder einem anderen biokompatiblen Material gebildet. Das erste Element 5 könnte aber auch ein Teil der Blutpumpe 3 bzw. ein Teil des Pumpenauslasses 72 sein, beispielsweise ein Auslassstutzen der Blutpumpe 3.

Bei dem Hohlkörper 79 des zweiten Elements 6 handelt es sich beispielsweise um eine implantierbare Kanüle, beispielsweise eine Gefäßprothese aus einem Graftmaterial, also ein Graft. Beispielsweise weist der Hohlkörper 79 eine schlauchförmige textile Trägerstruktur auf. Diese kann vor ihrer Verwendung mit einem dichtenden Material, wie etwa Rindergelatine, abgedichtet sein oder aber auch erst durch die Tränkung mit Blut abgedichtet werden (sogenanntes Glotting).

Das System 1 ist dazu eingerichtet, die beiden Elemente 5 und 6, also Hohlkörper 78, 79, lösbar und fluiddicht miteinander zu verbinden, so dass die beiden Fließkanäle 74, 75 einen durchgängigen Fließkanal bilden. Siehe beispielsweise Figuren 26A, 26B. Hierzu umfasst das System 1 einen ersten Konnektor 11 und einen zweiten Konnektor 12.

In Figuren 25B und 25C sind die beiden Konnektoren 11, 12 und die beiden Elemente 5, 6 miteinander verbunden. In Figur 25A sind die beiden Konnektoren 11, 12 und die beiden Elemente 5, 6 voneinander getrennt.

Wie in Figur 26A gezeigt ist, kann der erste Konnektor 11 als ein Rohrelement 73 ausgestaltet sein, das an dem zweiten Ende 8 des ersten Elements 5 angeordnet ist. (Das erste Element 5 ist in Figur 26A bis 29B nur teilweise dargestellt.) In dem gezeigten Beispiel ist der erste Konnektor 11 bzw. das Rohrelement 73 mit dem ersten Element 5 fest verbunden und beispielsweise einteilig mit dem ersten Element 5 ausgebildet. Prinzipiell kann der erste Konnektor 11 bzw. das Rohrelement 73 durch einen axialen Endabschnitt des ersten Elements 5 an dessen zweiten Ende 8 gebildet sein, an dem das erste Element 5 beispielsweise einen reduzierten Außendurchmesser aufweisen kann, wie in Figur 26A dargestellt ist.

Wie in Figur 26A außerdem dargestellt ist, sind der Außendurchmesser des ersten Konnektors 11 und der Innendurchmesser des zweiten Elements 6 derart aufeinander abgestimmt, dass der erste Konnektor 11 mit seinem vorderen Ende 15 in den Fließkanal 75 des zweiten Elements 6 axial eingeschoben werden kann.

Wie in Figuren 26A und 26B gezeigt ist, kann der zweite Konnektor 12 beispielsweise als ein Klemmelement 26 ausgestaltet sein, beispielsweise in Form einer Schelle 76 bzw. in Form einer geschlitzten Hülse. Der zweite Konnektor 12 ist typischerweise derart ausgestaltet, dass er das zweite Ende 10 des zweiten Elements 6 sowie das darin eingeschobene vordere Ende 15 des ersten Konnektors 15 umgreifen kann, um auf diese Teile eine Klemmkraft auszuüben. In diesem Fall kann der zweite Konnektor 12 bzw. das Klemmelement (Schelle) 76 in einen gespannten Zustand überführt werden, um eine radial nach innen auf das zweite Element 6 einwirkende Klemmkraft auszuüben und somit eine fluiddichte Klemmverbindung zwischen dem zweiten Element 6 und dem ersten Konnektor 11 herzustellen. Beispielweise weist der zweite Konnektor 12 (Klemmelement, Schelle 76) zur Herstellung und/oder zum Stabilisieren des gespannten Zustands einen Schraubverschluss mit einer Spannschraube 77 und einem korrespondierenden Gewindeteil auf. Alternativ könnte der zweite Konnektor 12 bzw. das Spannelement (Schelle) 76 auch einen Rastverschluss mit entsprechenden Rastelementen oder ein elastisches Federelement umfassen. Beispielsweise ist der zweite Konnektor 12 bzw. das Klemmelement (Schelle) 76 aus einem Metall und/oder einem Polymer gefertigt.

Das System 1 umfasst außerdem eine Sicherungshülse bzw. Hülse 13. Die Hülse 13 umfasst einen hülsenförmigen Hauptkörper 31 und ein Schutzelement 81. Das Schutzelement 81 bildet einen Knickschutz und einen Schneidschutz für das zweite Element 6 bzw. den zweiten Hohlkörper (Graft) 79.

Die Sicherungshülse 13 kann in diesem Beispiel über das erste Ende 9 des zweiten Element 6 auf das zweite Element 6 aufgeschoben und dann axial bis zum zweiten Ende 10 des zweiten Elements 6 verschoben werden. Siehe beispielsweise Figur 28. Durch entgegengesetztes axiales Verschieben kann die Sicherungshülse 13 prinzipiell auch wieder von dem zweiten Element 6 herunter geschoben und getrennt werden.

Wenn der erste Konnektor 11 mit dem zweiten Konnektor 12 verbunden ist, wie in Figuren 25B und 25C und in Figuren 28, 29A und 29B gezeigt ist, ist die Hülse 13 durch manuelles axiales Verschieben der Sicherungshülse 13 relativ zum ersten Konnektor 11 und dem zweiten Konnektor 12 in eine Sicherungsposition bewegbar. Diese Konfiguration ist in Figur 25C sowie in Figuren 29A und 29B gezeigt.

Wie in Figur 25C sowie in Figuren 29A und 29B gezeigt ist, nimmt die Sicherungshülse 13 in der Sicherungsposition den ersten Konnektor 11 und den zweiten Konnektor 12 jeweils in sich auf, in dem gezeigten Beispiel jeweils sogar vollständig, und überdeckt so den ersten und zweiten Konnektor 11, 12. Die Sicherungshülse 13 definiert zu diesem Zweck einen Hohlraum 14, der die Sicherungshülse 14 in axialer Richtung vollständig und durchgängig durchläuft. Der Hohlraum 14 verläuft durch den hülsenförmigen Hauptkörper 31 hindurch und wird in dem Schutzelement als Kanal 80 fortgesetzt.

Das Schutzelement 81 kann beispielsweise wie eines der in der europäischen Patentanmeldung EP 16 16 4527.0 vorgeschlagenen Schutzelemente ausgestaltet sein. Beispielsweise umfasst das Schutzelement 31 eine Mehrzahl von Segmenten 82, die in einer Abfolge nebeneinander angeordnet sind. Jedes der Segmente 82 definiert einen Teilabschnitt des Kanals 80 des Schutzelements 81. Die Segmente 82 sind aus einem relativ weichen Silikon gebildet (beispielsweise Shore A 30-90). Die Segmente 82 können beispielsweise Spritzgussteile sein. Der Hauptkörper 31 der Sicherungshülse 13 ist dahingegen beispielsweise aus einem relativ harten Silikon gebildet (vorzugsweise Shore B-C).

Das Schutzelement 81 weist mehrere (zum Beispiel drei) ebenfalls aus Silikon gebildete Verbindungselemente 83 auf, welche die Segmente 82 miteinander verbinden. Die Verbindungselemente 83 sind zu diesem Zweck mit den Segmenten 82 verklebt. Die Verbindungselemente sind in Form von Bändern ausgestaltet, bilden also flache, kabelförmige Elemente. Die Verbindungselemente 83 sind in einer Umfangsrichtung um das Schutzelement 81 voneinander beabstandet angeordnet. Der Hauptkörper 31 der Sicherungshülse 13 ist mit dem vordersten Segment 82 verklebt und somit mit dem Sicherungselement 31 als ganzes stoffschlüssig verbunden.

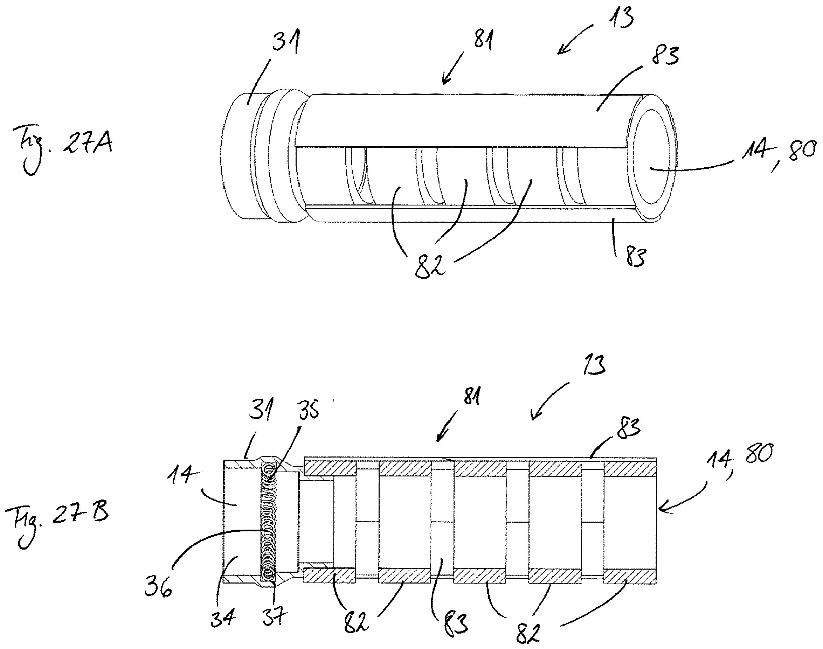

Das System 1 umfasst außerdem eine Rastvorrichtung mit einem Rastelement 35 und einem Gegenrastelement 41. Siehe beispielsweise Figuren 27B und 29B. Die Rastvorrichtung bzw. das Rastelement 35 und das Gegenrastelement 41 sind dazu ausgestaltet, zwischen der Sicherungshülse 13 und dem zweiten Konnektor 12 eine Rastverbindung herzustellen, wenn sich die Sicherungshülse relativ zum ersten Konnektor 11 und relativ zum zweiten Konnektor 12 in einer Sicherungsposition befindet. Diese Konfiguration ist in Figur 25C sowie in Figuren 29A und 29B gezeigt.

Die Sicherungshülse 13 weist auf einer inneren Oberfläche 34 des Hauptkörpers 31 eine Ringnut 37 auf, in der das genannte Rastelement 35 der Rastvorrichtung angeordnet ist. Siehe Figuren 27B und 29B. Das Rastelement 35 ist beispielsweise als ein elastisches Ringelement 36 ausgestaltet, beispielsweise in Form einer ringförmig gewickelten Spiralfeder, die in einer (kräftefreien) Ausgangsform beispielsweise einen elliptischen Querschnitt aufweist.

Als das Gegenrastelement 41 für das elastische Ringelement 36 weist der als Schelle 76 ausgebildete zweite Konnektor 12 auf seiner äußeren Oberfläche 43 eine korrespondierende Ringnut 45 auf. Siehe Figuren 26A und 26B sowie Figuren 28 und 29B. Die Rastverbindung zwischen der Sicherungshülse 13 und dem zweiten Konnektor 12 wird durch das Eintauchen des Rastelements 35 in das Gegenrastelement 41 bewirkt, wenn sich die Sicherungshülse 13 in der Sicherungsposition befindet. Siehe Figuren 29A und 29B. Hierdurch wird gleichzeitig auch das Sicherungselement 81 so fixiert, dass es einen Teil des zweiten Elements in dem Kanal 80 aufnimmt und somit vor Beschädigungen (beispielsweise durch ungewolltes Einschneiden mittels eines Skalpells) und ungewollten Verformungen (Knicken, Zusammendrücken) schützt. Diese Rastverbindung kann durch eine genügend große axiale Verschiebekraft hergestellt bzw. wieder gelöst, ohne dass eine Drehbewegung erforderlich ist.

### Bezugszeichenliste:

- 1: System
- 2: Blutpumpensystem
- 3: Blutpumpe
- 4: Steuervorrichtung
- 5: (erstes) Element
- 6: (zweites) Element
- 7: erstes Ende
- 8: zweites Ende
- 9: erstes Ende
- 10: zweites Ende
- 11: erster Konnektor
- 12: zweiter Konnektor
- 13: Sicherungshülse (Hülse)
- 14: Hohlraum
- 15: vorderes Ende
- 16: vorderes Ende
- 17: Gehäuse
- 18: Gehäuse
- 19: Knickschutz
- 20: Knickschutz
- 21: hinteres Ende
- 22: hinteres Ende
- 23: Kupplungselement
- 24: Aufnahmeteil
- 25: Aufnahmebereich
- 26: Kontaktelemente
- 27: Riegelelement
- 28: Rastelement
- 29: Gelenk
- 30: Betätigungselement
- 31: Hauptkörper
- 32: Stützfläche
- 33: Stützfläche
- 34: Oberfläche
- 35: Rastelement
- 36: Ringelement
- 37: Ringnut
- 38: Oberfläche
- 39: Grifffläche
- 40: Gegenrastelement
- 41: Gegenrastelement
- 42: Oberfläche
- 43: Oberfläche
- 44: Ringnut
- 45: Ringnut
- 46: Steigfläche
- 47: Steigfläche
- 48: Anschlag
- 49: Anschlag
- 50: Markierung
- 51: Markierung
- 52: Rastelement
- 53: Rastelement
- 54: Vertiefung
- 55: Vertiefung
- 56: Ausbuchtung
- 57: Ausbuchtung
- 58: vorderes Ende
- 59: Ausbuchtung
- 60: Ausbuchtung
- 61: Anschlag
- 62: Rastarm
- 63: Rastzahn
- 64: Griffteil
- 65: Stufe
- 66: Entriegelungssteigfläche
- 67: Steigfläche
- 68: Haltefläche
- 69: Träger
- 70: hinteres Ende
- 71: Ausbuchtung
- 72: Pumpenauslass
- 73: Rohrelement
- 74: Fließkanal
- 75: Fließkanal
- 76: Klemmelement
- 77: Spannschraube
- 78: Hohlkörper
- 79: Hohlkörper
- 80: Kanal
- 81: Schutzelement
- 82: Segment
- 83: Verbindungselement

## Patentansprüche

1. System (1) zum Sichern einer lösbaren Verbindung zwischen zwei Elementen (5, 6), insbesondere zwischen zwei Kabeln oder zwischen zwei Hohlkörpern, umfassend:
einen ersten Konnektor (11) und einen zweiten Konnektor (12), der mit dem ersten Konnektor (11) lösbar verbindbar ist,
eine Sicherungshülse (13), die, wenn der erste Konnektor (11) mit dem zweiten Konnektor (12) verbunden ist, durch Verschieben der Sicherungshülse (13) relativ zum ersten Konnektor (11) und relativ zum zweiten Konnektor (12) in eine Sicherungsposition bewegbar ist, in der die Sicherungshülse (13) den ersten Konnektor (11) und den zweiten Konnektor (12) vollständig oder zumindest teilweise in sich aufnimmt, und
eine Rastvorrichtung mit mindestens einem Rastelement (35, 52, 53),
wobei die Rastvorrichtung ausgestaltet ist, zwischen der Sicherungshülse (13) in der Sicherungsposition und dem ersten Konnektor (11) und/oder dem mit dem ersten Konnektor (11) verbundenen zweiten Konnektor (12) eine Rastverbindung herzustellen.

2. System (1) nach Anspruch 1, **dadurch gekennzeichnet, dass** mindestens ein Rastelement (35, 52, 53) des mindestens einen Rastelements (35, 52, 53) der Rastvorrichtung ausgestaltet ist,
- auf einer Oberfläche (42) des ersten Konnektors (11) einzurasten und/oder
- auf einer Oberfläche (43) des zweiten Konnektors einzurasten und/oder
- auf einer Oberfläche (34) der Sicherungshülse (13) einzurasten, wenn sich die Sicherungshülse (13) in der Sicherungsposition befindet.

3. System (1) nach einem der vorangehenden Ansprüche, **dadurch gekennzeichnet, dass** die Sicherungshülse (13) durch Verschieben der Sicherungshülse (13) relativ zum ersten Konnektor (11) und/oder relativ zum zweiten Konnektor (11) in eine von der Sicherungsposition verschiedene Halteposition relativ zum ersten Konnektor (11) und/oder relativ zum zweiten Konnektor (12) bewegbar ist, wobei die Rastvorrichtung vorzugsweise ausgestaltet ist, zwischen der Sicherungshülse (13) in der Halteposition und dem ersten Konnektor (11) oder dem zweiten Konnektor (12) eine Rastverbindung herzustellen.

4. System (1) nach einem der vorangehenden Ansprüche, **dadurch gekennzeichnet, dass** mindestens ein Rastelement (35, 52, 53) des mindestens einen Rastelements (35, 52, 53) der Rastvorrichtung mit
- dem ersten Konnektor (11) oder
- dem zweiten Konnektor (12) oder
- der Sicherungshülse (13)
fest verbunden ist.

5. System (1) nach einem der vorangehenden Ansprüche, **dadurch gekennzeichnet, dass** mindestens ein Rastelement (35, 52, 53) des mindestens einen Rastelements (35,52,53) der Rastvorrichtung als ein elastisches Ringelement (36) ausgestaltet ist, vorzugsweise als eine ringförmig gewickelte Spiralfeder oder als ein Axialsicherungsring oder als ein O-Ring.

6. System (1) nach einem der vorangehenden Ansprüche, **dadurch gekennzeichnet, dass** mindestens ein Rastelement (52, 53) des mindestens einen Rastelements (52, 53) der Rastvorrichtung eine Ausbuchtung (56, 57) aufweist, die von einer inneren Oberfläche (34) der Sicherungshülse (11) ausgehend radial nach innen vorspringt, wobei die Ausbuchtung (56, 57) des mindestens einen Rastelements (52, 53) vorzugsweise als eine Noppe, als eine Rippe oder als ein Steg ausgestaltet ist.

7. System nach einem der Anspruch 6, **dadurch gekennzeichnet, dass** die Sicherungshülse (13) einen hülsenförmigen Hauptkörper (31) umfasst, wobei die mindestens eine Ausbuchtung (56, 57) weicher als der hülsenförmige Hauptkörper (31) der Sicherungshülse ist.

8. System (1) nach einem der vorangehenden Ansprüche, **dadurch gekennzeichnet, dass** zwei Rastelemente (52) des mindestens einen Rastelements (52) der Rastvorrichtung jeweils eine Ausbuchtung (56) aufweisen, die von einer inneren Oberfläche (34) der Sicherungshülse (13) radial nach innen vorspringen, wobei die beiden Ausbuchtungen (56) in einem Ausgangszustand der Sicherungshülse (13) einen ersten Abstand voneinander einnehmen, wobei der erste radiale Abstand in einer ersten radialen Richtung definiert ist und derart bemessen ist, dass die beiden Ausbuchtungen (56) in einer Oberfläche (42) des ersten Konnektors (11) oder einer Oberfläche (43) des zweiten Konnektors (12) eingerastet sind, wenn sich die Sicherungshülse (13) im Ausgangszustand relativ zu dem ersten Konnektor (11) und dem mit dem ersten Konnektor (11) verbundenen zweiten Konnektor (12) in der Sicherungsposition befindet, wobei die Sicherungshülse (13) durch eine in einer zweiten radialen Richtung, die von der ersten radialen Richtung verschieden ist, auf die Sicherungshülse (13) einwirkende radiale Kompressionskraft in einen Zwischenzustand verformbar ist, wobei die beiden Ausbuchtungen (56) in dem Zwischenzustand der Sicherungshülse (13) einen in der ersten radialen Richtung definierten zweiten Abstand voneinander einnehmen, wobei der zweite Abstand größer als der erste Abstand ist und derart bemessen ist, dass die beiden Ausbuchtungen (56) in der Oberfläche (42) des ersten Konnektors oder der Oberfläche (43) des zweiten Konnektors (12) nicht eingerastet sind, wenn sich die Sicherungshülse (13) im Zwischenzustand relativ zu dem ersten Konnektor (11) und dem mit dem ersten Konnektor (12) verbundenen zweiten Konnektor (12) in der Sicherungsposition befindet.

9. System (1) nach einem der vorangehenden Ansprüche, **dadurch gekennzeichnet, dass** die Rastvorrichtung mindestens einen flexiblen Rastarm (62) umfasst.

10. System (1) nach einem der vorangehenden Ansprüche, **dadurch gekennzeichnet, dass** der erste Konnektor (11) und/oder der zweite Konnektor (12) mindestens ein Riegelelement (27) aufweist, wobei das mindestens eine Riegelelement (27) ausgestaltet ist, die lösbare Verbindung zwischen dem ersten Konnektor (11) und dem zweiten Konnektor (12) zu verriegeln.

11. System (1) nach einem der vorangehenden Ansprüche, **dadurch gekennzeichnet, dass**
- der erste Konnektor (11) als ein Stecker ausgestaltet ist und der zweite Konnektor (12) als eine Kupplung oder Buchse ausgestaltet ist, oder dass der erste Konnektor (11) als eine Kupplung oder Buchse ausgestaltet ist und der zweite Konnektor (12) als ein Stecker ausgestaltet ist, oder
- der erste Konnektor (11) als ein Rohrelement (73) und der zweite Konnektor (12) als ein Spannelement (76) ausgestaltet ist oder dass der zweite Konnektor (12) als ein Rohrelement (73) und der erste Konnektor (11) als ein Spannelement (76) ausgestaltet ist, wobei das Spannelement (76) ausgestaltet ist, das Rohrelement (73) zu umgreifen und auf das Rohrelement (73) eine Klammkraft auszuüben.

12. System (1) nach einem der vorangehenden Ansprüche, **dadurch gekennzeichnet, dass** das System (1) ein formflexibles Schutzelement (31) für das erste Element (5) und/oder für das zweite Element (6) umfasst, wobei das Schutzelement (81) mit der Sicherungshülse (13) verbunden ist und einen Kanal (80) bildet, durch den hindurch das erste Element (5) und/oder das zweite Element (6) zumindest abschnittsweise hindurch verläuft, wenn sich die Sicherungshülse (13) in der Sicherungsposition befindet, wobei das Schutzelement (31) vorzugsweise eine Mehrzahl von Segmenten (82) umfasst, die in einer Abfolge nebeneinander angeordnet sind und jeweils einen Teilabschnitt des Kanals (80) des Schutzelements (81) definieren.

13. System (1) nach Anspruch 12, **dadurch gekennzeichnet, dass** die Sicherungshülse (31) einen hülsenförmigen Hauptkörper (31) umfasst, der fest mit dem Schutzelement (81) verbunden ist und der mindestens ein Rastelement (35) des mindestens einen Rastelements der Rastvorrichtung aufweist, wobei das Rastelement (35) des hülsenförmigen Hauptkörpers (31) vorzugsweise als ein elastisches Ringelement (36) ausgestaltet ist, vorzugsweise als eine ringförmig gewickelte Spiralfeder.

14. System (1) nach einem der vorangehenden Ansprüche, **dadurch gekennzeichnet, dass** das erste Element (5) und das zweite Element (6) jeweils als ein Hohlkörper (78, 79) ausgebildet sind und jeweils einen Fließkanal (74, 75) zum Leiten einer Flüssigkeit bilden, vorzugsweise zum Leiten von Blut, wobei der erste Konnektor (11) und der zweite Konnektor (12) dazu ausgestaltet sind, die beiden Hohlkörper (78, 79) fluiddicht miteinander zu verbinden, so dass deren Fließkanäle (74, 75) einen durchgängigen Fließkanal bilden.

15. Blutpumpensystem (2), umfassend eine Blutpumpe (3) und ein System (1) nach einem der vorangehenden Ansprüche.

16. Blutpumpensystem (2) nach Anspruch 15, **dadurch gekennzeichnet, dass** das Blutpumpensystem (2) außerdem umfasst:
- eine Steuervorrichtung (4) für die Blutpumpe (3),
- ein pumpenseitiges Kabel mit einem ersten Ende (7), das mit der Blutpumpe (3) verbindbar ist, und einem zweiten Ende (8),
- ein steuerseitiges Kabel mit einem ersten Ende (9), das mit der Steuervorrichtung (4) verbindbar ist, und einem zweiten Ende (10), wobei der erste Konnektor (11) des Systems (1) mit dem zweiten Ende (8) des pumpenseitigen Kabels fest verbunden ist und der zweite Konnektor (12) des Systems (1) mit dem zweiten Ende (10) des steuerseitigen Kabels fest verbunden ist.

17. Blutpumpensystem (2) nach Anspruch 15, **dadurch gekennzeichnet, dass** das Blutpumpensystem (2) außerdem umfasst:
- einen ersten Hohlkörper (78) mit einem ersten Ende (7), das mit der Blutpumpe (3) verbunden oder verbindbar ist, und mit einem zweiten Ende (8), wobei der erste Hohlkörper (78) einen ersten Fließkanal (74) zum Leiten von Blut bildet,
- einen zweiten Hohlkörper (79) mit einem ersten Ende (9) und einem zweiten Ende (10), der einen zweiten Fließkanal (74) zum Leiten von Blut bildet,
wobei der erste Konnektor (11) des Systems (1) mit dem zweiten Ende (8) des ersten Hohlkörpers (78) verbindbar ist und der zweite Konnektor (12) des Systems (1) mit dem zweiten Ende (10) des zweiten Hohlkörpers (79) verbindbar ist, wobei die Konnektoren (11, 12) ausgestaltet sind, die beiden Hohlkörper (78, 79) fluiddicht miteinander zu verbinden, so dass deren Fließkanäle (74, 75) einen durchgängigen Fließkanal bilden.
